# EUROPEAN PATENT SPECIFICATION

(11) **EP 1 968 664 B1**
(45) Date of publication and mention of the grant of the patent: **27.10.2010**
(21) Application number: 06838790.1
(22) Date of filing: 01.12.2006
(51) Int. Cl.: A61L 33/00, A61L 33/04

(54) **POLYMER COMPOSITIONS, COATINGS AND DEVICES, AND METHODS OF MAKING AND USING THE SAME**
POLYMERZUSAMMENSETZUNGEN, ÜBERZÜGE UND VORRICHTUNGEN UND HERSTELLUNGS- UND ANWENDUNGSVERFAHREN DAMIT
COMPOSITIONS POLYMERES, REVETEMENTS ET DISPOSITIFS, ET PROCEDES DE FABRICATION ET D'UTILISATION DE CEUX-CI

(30) Priority: 02.12.2005 US 741601 P
(43) Date of publication of application: 17.09.2008
(73) Proprietor: THE REGENTS OF THE UNIVERSITY OF MICHIGAN, Ann Arbor, MI 48109-1280 (US); Michigan Critical Care Consultants, Inc., Ann Arbor, MI 48103 (US)
(72) Inventor: MEYERHOFF, Mark, E., Ann Arbor, MI 48109 (US); CHA, Wansik, Ann Arbor, MI 48109 (US); HWANG, Sang-Yeul, Ann Arbor, MI 48109 (US); REYNOLDS, Melissa, M., Ann Arbor, MI 48103 (US)
(74) Representative: Walker, Ross Thomson
(86) International application number: PCT/US2006/046013
(87) International publication number: WO 2007/064895

(56) References cited:
- WO-A-96/35416
- US-A1- 2002 115 559
- US-A1- 2003 235 602
- US-A1- 2005 008 676
- W. CHA AND M.E. MEYERHOFF: "S-Nitrosothiol Detection via Amphoteric Nitric Oxide Sensor with Surface Modified Hydrogel Layer Containing Immobilized Organiselenium Catalyst" LANGMUIR, vol. 22, 28 September 2006 (2006-09-28), pages 10830-10836, XP002464682
- W. CHA AND M.E. MEYERHOFF: "Catalytic generation of nitric oxide from S-nitrosothiol using immobilized organoselenium species" BIOMATERIALS, vol. 28, 7 September 2006 (2006-09-07), pages 19-27, XP002464683
- FROST M C ET AL: "Preparation and characterization of implantable sensors with nitric oxide release coatings" MICROCHEMICAL JOURNAL, NEW YORK, NY, US, vol. 74, no. 3, June 2003 (2003-06), pages 277-288, XP002325227 ISSN: 0026-265X
- Z. DONG ET AL: "Aryl Thiol Substrate 3-Carboxy-4-Nitrobenzenethiol Strongly Stimulating Thiol Peroxidase Activity of Glutathione Peroxidase Mimic 2,2'-Ditellurobis(2-Deoxy-beta-Cyclodextri n)" JOURNAL OF THE AMERICAN CHEMICAL SOCIETY, vol. 126, 24 November 2004 (2004-11-24), page 16395-16404, XP002464685
- Y. HOU ET AL: "Seleno Compounds and Glutathione Peroxidase Catalyzed Decomposition of s-Nitrosothiols" BIOCHEMICAL AND BIOPHYSICAL RESEARCH COMMUNICATIONS, vol. 228, 1996, pages 88-93, XP002464686

## Description

### BACKGROUND

Blood-contacting and implantable medical devices such as vascular grafts, intravascular catheters, coronary artery and vascular stents, insulation materials for electrical leads of pacemakers and defibrillators, extracorporeal bypass circuits, and oxygenators, etc. are manufactured from many different materials. The incompatibility of these materials with human blood and tissue may cause serious complications in patients, and ultimately functional device failure. Implantation of such devices into blood vessels may cause damage to the endothelial layers and an almost immediate inflammatory response throughout the implant site. For example, in addition to opening the artherosclerotically obstructed artery, placement of a vascular stent may, in some instances, cause endothelial disruption, fracture of internal lamina and dissection of the media of the diseased vessel. Within three to seven days post injury, several processes may occur including adhesion, and the recruitment and activation of neutrophils, monocytes and lymphocytes in an attempt to destroy the foreign body.

Blood compatible biomaterials are generally developed using one of two approaches. The first method utilizes chemical surface moieties which suppress blood-material interactions. The second method attempts to mimic natural endothelial cells (EC) which line the inner walls of all healthy blood vessels. Endothelial cells generate nitric oxide (NO) which inhibit platelet function and smooth muscle cell proliferation. Materials that include such properties may also be important for the treatment of circulatory diseases.

It may be desirable to provide materials, especially materials for use with medical devices, that can generate nitric oxide *in vivo* and/or provide anti thrombogenic properties of nitric oxide in a medical device. Such devices may, for example, obviate or minimize the need to administer anticoagulants, which may have clinical risks such as excess bleeding.
US 2002/115559A1 (Batchelor M. et al) discloses biocompatible materials that have the ability to release nitric oxide *in situ* at the surface-blood interface when in contact with blood. The materials which may be polymers, metals, carbon and the like are provided with biocatalysts or biomimetic catalysts on their surface that have nitrate, nitrate and/or nitrosothiol-reducing capability.

### SUMMARY

The present disclosure is directed in part to compositions according to claim 1 that are capable of generating nitric oxide, e.g., *in-vivo.* The composition includes a chalcogenide compound or moiety, and further includes a biocompatible matrix. Chalcogenide compounds are selected from organoselenium, and organotellurium.
Chalcogenide compounds may also be enzymes that include at least one of selenium, or tellurium.

A biocompatible, thromboresistant coating for use on an implantable medical device is provided herein that includes a chalcogenide compound that induces nitric oxide formation; and a biocompatible matrix incorporating said chalcogenide compound. The biocompatible matrix may include a polymer, which may for example, be hydrophilic. In other embodiments, the matrix may comprise a polymer that includes one or more of: a carboxyl moiety, an aldehyde moiety, or a halide moiety. In a non-limiting example, the matrix includes more than about 0.6 mmol/g carboxyl moieties. Chalcogenide compounds may include a carboxyl and/or amine moiety.

Chalcogenide compounds are covalently bound to the matrix, e.g., to a polymer, a porous membrane structure, a fibrous matrix, or fumed silica. The coatings and matrices disclosed herein may further include a therapeutic agent.

### BRIEF DESCRIPTION OF THE FIGURES

The embodiments and practices of the present disclosure, other embodiments, and their features and characteristics, will be apparent from the description, drawings and claims that follow.

Figure 1 depicts a hypothetical catalytic cycle between S-nitrosthiols (RSNO) and selenol compounds;

Figure 2 depicts exemplary organo selenium compounds;

Figure 3 depicts the quantitative response of a RSNO sensor that contains ebselen, at various concentrations of SNAP (S-nitroso-N-acetyl-DL-pencillamine) between 0.5 µm and 25 µm;

Figure 4 pictorially represents one possible reaction mechanism to create Chelex particles bound on the surface of SeCA;

Figure 5 shows SNAP decomposition by SeCA-immobilized Chelex particles;

Figure 6 depicts the immobilization of selenocystamine on filter paper;

Figure 7 shows NO generation from SeCA-immobilized on 0.5 cm² filter paper;

Figure 8 shows the production of NO from Se-immobilized filter paper;

Figure 9A shows a response curve of the Se-based RSNO sensor response to various RSNO species;

Figure 9B shows a schematic view of the Se-based RSNO sensor, where the sensor is constructed by mounting Se-immobilized filter paper on an amperometric NO sensor;

Figure 10 depicts NO current changes from oxygen reduction by Se-FP;

Figure 11 shows a reaction scheme for the preparation of a diorgano telluride compound;

Figure 12A depicts a hypothetical catalytic cycle between RSNO and tellurol compounds;

Figure 12B depicts a hypothetical catalytic cycle for diaromatic ditelluride compounds;

Figure 13 shows the NO generation profile monitored by NOA when 0.025 limo of diorgano ditelluride (RTeTeR) is added into 0.2 µmol of S-nitrosoglutathione (GSNO) and 1.0 µmol of glutathione (GSH) in 2ml of PBS buffer (pH 7.4) in the presence of 1.0 µmol of EDTA at room temperature (RT);

Figure 14A depicts a synthetic route to form selenium derivatized fumed particles;

Figure 14B shows the NO generation curve from selenium derivatized particles in PBS buffer with standard injection of S-nitroso-N-acetyl-penicillamine; .

Figure 15A is a schematic representation of polymer structures of Se-immobilized , polethylenimine (RSePEI);

Figure 15B depicts an amperometric detection scheme of a RSNO sensor based on a catalytic RSe-hydrogel layer;

Figure 16 depicts the catalytic NO generation by a piece of RSePEI immobilized on dialysis membranes (DM) (RSePEI-DM) (0.5 cm²) in a PBS (pH 7.3) solution containing 0.5 mM EDTA, 100 µM GSNO and 50 µM GSH at room temperature, (Note that the given DM is inserted into ( ↓) or removed from ( ↑) the solution at each arrow point indicated);

Figure 17 depicts endogenous RSNO detection with two amperometric NO sensors, one is a control NO sensor with control DM, the other is a RSNO sensor with RSePEI-DM, the measurement is conducted at 25 °C in a water bath with PBS (pH 7.3);

Figure 18 depicts a reaction scheme for 5,5'-ditelluro-2,2'-dithiophenecarboxylic acid (DTDTCA) and its tellurosulfide polymer 7;

Figures 19A, 19B and 19C each depict measurements of catalytic NO generation by ditelluride compounds, where the arrows indicate addition of a given species into the mixure, 19A and 19B respectively depict the measurements of catalytic NO generation by 2.5 µM 5,5'-ditelluro-2,2'-dithiophenecarboxylic acid (DTDTCA) 2 in a solution of (A) 25 µM GSNO and 100 µM GSH, and (B) 50 µM GSNO and 100 µM GSH in PBS buffer, pH 7.4 (0.5 mM EDTA) via a chemiluminescence NO analyzer (NOA), and 19C depicts Tellurosulfide polymer 7-mediated catalytic NO generation from 100 µM GSNO and 25 µM GSH in PBS buffer;

Figure 20 depicts the synthesis of interpenetrating networks of hydro gels 3 (Te linked polymer, A) and 4 (blank polymer B); and

Figure 21 depicts the nitric oxide flux induced by (A) hydrogel 3 and (B) hydrogel 4 upon immersion/removal into the solution of 100 µM GSH and 100 µM GSNO in deoxygenated 2 ml of 10 mM PBS buffer (pH=7.4) containing 0.5 mM EDTA at RT and measured by a chemiluminescence nitric oxide analyzer (NOA).

### DETAILED DESCRIPTION

This disclosure is directed, at least in part, to biocompatible compositions that may be suitable for use with, a bioimplant. Compositions are provided that include a chalcogenide compound, selected from organoselenium compounds, and organotellurium compounds, covalently bound to a matrix (one non-limitative matrix (one non-limitative example of which is a polymer).

This disclosure also provides, in part, a composition for nitric oxide formation, which includes a chalcogenide compound, wherein the chalcogenide compound includes a moiety selected from an organoselenium moiety, and an organotellurium moiety and combinations thereof; and/or an enzyme including at least one of selenium, or tellurium.

Exemplary compounds include di-selenium compounds and di-tellurium compounds.

The composition may further include a biocompatible and/or a biodegradable matrix, such as a biocompatible and/or biodegradable polymer. Such polymers may be hydrophilic or hydrophobic. In some embodiments, the matrix includes a polymer that has one or more of: a carboxyl moiety, an aldehyde moiety, an amine moiety, or a halide moiety. In other embodiments, the matrix or polymer includes more than about 0.6 mmol/g carboxyl moieties. Alternatively, the matrix may include a porous membrane structure.

Compositions of the present disclosure include chalcogenide compounds that include a carboxyl moiety and/or an amine moiety.

The chalcogenide compound is covalently bound to the polymer and/or matrix. The chalcogenide moiety or compound includes an organoselenium moiety or an organotellurium moiety. In some embodiments, an organoselenium moiety is selected from selenocystamine, selenocystine, 3,3'-diselenodipropionic acid, selenocysteine, ebselen, propyl-selenocystine, allyl-selenocystine, methyl-selenocystine, selenomethionine, selenium choline, and combinations thereof. In other embodiments, a composition may include selenium enzymes, such as glutathione peroxidase and thiredoxin reductase.

A composition is also provided that includes a matrix covalently bound, directly or through a chemical moiety, to a chalcogenide moiety, wherein the chalcogenide moiety is selected from an organoselenium moiety, an organotellurium moiety, and/or an enzyme including at least one of selenium, or tellurium.

In some embodiments, the chalcogenide moiety may include a moiety selected from structure I or II:

R₁-R₂-R₃-A-R₄ I

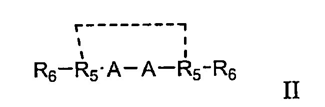

wherein R₁ represents an H, alkyl, aryl or a bond; R₂ represents an alkyl, amido, carboxyl, amino, or a bond; R₃ represents an alkyl or a bond; A represents independently for each occurrence Se, or Te; R₄ represents an H or alkyl; the dashed line represents an optional bond included if structure II is cyclic; R₅ represents independently for each occurrence, an alkyl, aryl, amido, carboxyl, amino, or a bond; and R₆ represents independently for each occurrence an H, carboxyl, amino, aryl, or a bond.

Matrices include for example polyurethane, polyester, polyethyleneimine, polymethacrylate, polytetrafluoroethylene, and polydimethylsiloxane. The matrix may include immobilization moieties attached thereto. Such immobilization moieties may include, but are not limited to, particles, a fibrous matrix such as cellulose, or nano- or micro-particles including cellulose, and fumed silica. Fumed silica derivatized with a chalcogenide may be used as a polymer filler, e.g., may form part of a composition that includes a filled polymer, for example, a filled polyurethane.

Coatings for medical devices that generate nitric oxide are contemplated herein. Such coatings may include a composition of a polymer and a chalcogenide compound. Such coatings may be layered, e.g., may include a first polymer layer having a chalcogenic compound therein, and a second polymer layer. The second polymer layer may have hydrophilic properties and/or may be biodegradable. In some embodiments, the second polymer layer may also include another therapeutic agent.

For example, the coating may include a first layer of polyethylenimine covalently bound to a selenium moiety. Such a first layer may be in, e.g., the form of a hydrogel. The coating may then further include a second layer of polytetrafluoroethylene with a therapeutic agent, e.g., an anti-infective agent.

Also provided herein is an analyte sensor including an electrode surface; and a biocompatible analyte-permeable composition or coating as disclosed herein. The coating may be disposed on the electrode surface of the analyte sensor. It is believed that incorporating the chalcogenide compound may improve, for example, the biocompatibility of the implantable sensors.

Compositions disclosed herein may be administered locally, e.g., at a site of device implantation, to deliver nitric oxide directly. Local administration of the composition may be via a suture, a vascular implant, a stent, a heart valve, a drug pump, a drug delivery catheter, an infusion catheter, a drug delivery guidewire or an implantable medical device. For example, nitric oxide may be delivered directly to a local site by implanting a medical device coated with compositions and/or coatings disclosed herein.

In one aspect of this disclosure, a method for direct delivery of nitric oxide to a targeted site in a patient in need thereof is provided, including administering a composition of the present disclosure directly to the targeted site in the patient.

In another aspect of this disclosure, a medical device is disclosed that includes an embodiment of the composition disclosed herein. Such a medical device includes, but is not limited to an intravascular or extravascular medical device, a balloon, a catheter tip, a prosthetic heart valve, a suture, a surgical staple, a synthetic vessel graft, a stent, a stent graft, a vascular or non-vascular graft, a shunt, an aneurysm filler, an intraluminal paving system, a guide wire, an embolic agent, a filter, a drug pump, an arteriovenous shunt, an artificial heart valve, an artificial implant, a foreign body introduced surgically into the blood vessels or at a vascular or non-vascular site, a lead, a pacemaker, an implantable pulse generator, an implantable cardiac defibrillator, a cardioverter defibrillator, a defibrillator, a spinal stimulator, a brain stimulator, a sacral nerve stimulator, a chemical sensor, a breast implant, an interventional cardiology device, a catheter, plastic tubing, or a dialysis bag or membrane.

Also provided is a method for inhibiting platelet aggregation and platelet adhesion caused by the exposure of blood to a medical device. Such a method includes incorporating at least one composition as disclosed herein into or on the medical device prior to exposing the medical device to blood. In another embodiment, a method for treating an injured tissue in a patient in need thereof includes administering at least one composition disclosed herein, to the site of the injured tissue in the patient. A method of promoting angiogenesis in a subject afflicted with atherosclerosis is also provided, where the method includes implanting a composition disclosed herein to a subject at a tissue locus experiencing or at risk of insufficient blood perfusion.

A method of detecting nitrosothiols in a fluid is also provided, wherein the method includes contacting the fluid with a composition disclosed herein.

In some embodiments, a composition is provided that includes a compound represented by formula III, IV or V: 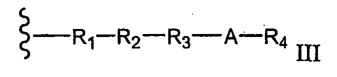 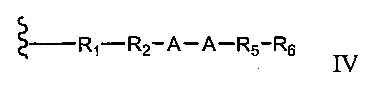 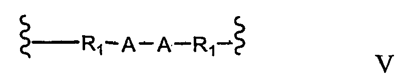 wherein 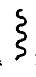 represents a polymer residue or a fibrous matrix; wherein R₁ represents an alkyl, aryl, carboxyl, or a bond; R₂ represents an alkyl, aryl amido, carboxyl, amino, or a bond; R₃ represents an alkyl or a bond; A represents independently for each occurrence Se, or Te; R₄ represents an H, aryl, or alkyl; R₅ represents an aryl, or alkyl and R₆ represents an H, carboxyl, or amino.

### Chalcogenide Compounds and Moieties

A variety of chalcogenide compounds and moieties are contemplated as being within the purview of the present disclosure. In some embodiments, such compounds may generate nitric oxide when in contact with a bodily fluid, for example, blood. Practitioners of the art will readily appreciate the circumstances under which various chalcogenide compounds are appropriate for use in the various compositions and devices disclosed herein.

"Chalcogen compounds" refer to compounds and moieties that include atoms within column 6A of the periodic table. Group 6A or chalcogen compounds may also be referred to as Group 16 compounds. Group 6A atoms include at least one of: oxygen, sulfur, selenium, tellurium, and polonium. "Chalcogenide compounds" refer to compounds and moieties that include heavier Group 6A atoms, and include at least one of: sulfur, selenium, tellurium, and polonium. Both unsubstituted chalcogen and chalcogenide compounds and substituted chalcogen and chalcogenide compounds are contemplated by the terms "chalcogenide compounds" and "chalcogen compounds." Substituted chalcogenide compounds refer to compounds and moieties having substituents replacing a hydrogen on one or more carbons of a hydrocarbon backbone. Such substituents may include, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety.

Without being limited to any theory, it is believed that chalcogenide compounds (such as substituted or unsubstituted organoselenium, organotellurium, organosulfur compounds, and combinations thereof); and/or enzymes including selenium, sulfur, tellurium, and combinations thereof, when exposed to endogenous or exogenous sources of nitrates, nitrites, or nitrosothiols (optionally in the presence of reducing agents), generate nitric oxide and/or an active species that generates NO within and/or at the surface of a composition. It is to be understood that the sources of nitrates, nitrites, nitrosothiols and reducing agents may be from bodily fluids such as blood, within the composition, within a device, and/or may be injected intravenously or otherwise added or administered to the bodily fluid of interest. Such reducing agents may include biochemical or organic reducing agents such as ascorbic acid, NADH, NADPH, thiol compounds such as glutathione, cysteine, dithreitol, 2-mercaptoethanol, 2-mercaptoethylamine, tris[2-carboethyl]phosphine hydrochloride, and the like. As used herein, the term "nitric oxide" or "NO" encompasses uncharged nitric oxide and charged nitric oxide species, including for example, nitrosonium ion and nitroxyl ion.

Such chalcogenide compounds may include alkylthio compounds and moieties, thiocarbonyl moieties, and selenoalkyl compounds. Exemplary organoselenium compounds include selenocystamine, selenocystine, 3,3'-diselenodipropionic acid, selenocysteine, ebselen, propyl-selenocystine, allyl-selenocystine, methyl-selenocystine, selenomethionine, and selenium choline. In some embodiments, organoselenium and/or organotellurium compounds may be diselenium or ditellurium compounds, e.g., comprise an -Se-Se- moiety and/or a -Te-Te- moiety.

Selenium compounds also encompass enzymes including selenium. Exemplary enzymes include glutathione peroxidase and thiredoxin reductase.

Sulfur compounds include organosulfur compounds, such as 2-mercaptoethanol, dithiothreitol, 2-mercaptoethylamine-HCl, cystamin, 2-aminoethyl-2-aminoethanethiolsulfonate, 3-mercaptopropionic acid, 2-(trimethylsilyl)ethanethiol, (3-mercaptopropyl)trimehoxysilane and sulfur-containing amino acids, peptides and their derivatives, such as cysteine and cystine, glucose-cysteine, N-isobutyrylcysteine, 2,3-dimercaptosuccinic acid-cysteine (1-2) mixed disulfide, and peptides containing cysteine residue, enzymes, proteins or their derivatives modified or synthesized to have S-moieties, for example, albumin and albumin-Cys, and polymers containing the above-mentioned S-containing molecules in their backbone or side-chains.

Tellurium compounds may include organotellurium compounds. Tellurium compounds may further include sulfur and/or selenium. Exemplary tellurium compounds include those ditellurium compounds that can be represented by the formula:

Ar-Te-Te-Ar

where Ar is a substituted or unsubstituted aryl moiety, such as for example, a heterocycle. A particular exemplary tellurium compound contemplated herein can be represented by the following formula:

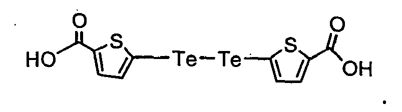

Mixed tellurium-selenium, mixed selenium-thiol, or mixed tellurium-thiol species are also contemplated by this disclosure. For example, compounds such as R-Se-Te-R, R-Se-S-R, R-Te-S-R, are provided, wherein each R represents independently an organic moiety. In some embodiments, such species will yield, after reduction, one or more selenol, thiol, or tellelanol species that are catalytic.

Organoselenium compounds may further include sulfur. For example, organoselenium compounds such as molecules, enzymes, proteins or polymers, or their composites can include sulfhydryl, disulfide, and selenosulfide functional moieties, as well as selenol/selenolate and diselenide moieties. In some embodiments, sulfur-containing moieties may stabilize catalytic sites by forming, for example, a selenosulfide bridge. Such a bridge may be reversibly cleavable to produce catalytic sites on an organoselenium compound.

Selenosulfide bonds may form as intermediate state during redox reactions. if S-moieties exist in proximity of Se-sites, for example, by coupling the above-mentioned species in a Se-immobilized polymer matrix. Compounds that include such bonds are contemplated by this disclosure. Such compounds include glutathione-glutaselenone (GS-SeG), Cys-Sec (a selenosulfide, Cys-cysteine, Sec-selenocysteine), Glutathione peroxidase-Se-SG (or a compound containing a -Cys, selenosulfide linkage), and polymers that include such a linkage, e.g., Polymer----Se-S-----Polymer.

Further chalcogenide compounds and moieties include those with the structure: 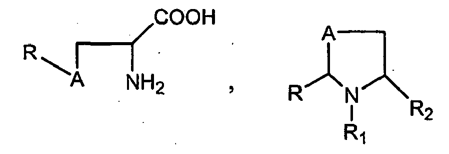 where R is any organic moiety, such as a drug, saccharide, or other moiety; R1 is selected from: an alkyl, amino, amido, carboxyl, or hydrogen; R₂ is selected from a carboxyl, alkoxy, alkyl, amino, or H; and A is selenium, sulfur, or tellurium.

In some embodiments, the chalcogenide compounds or moieties have been reduced by a reducing agent such as a borohydride (non-limiting examples of which include sodium borohydride, sodium cyanoborohydride, zinc borohydride) or other hydrides such as lithium aluminumhydride and butyl tinhydride, and diimide. Such reduced moieties are contemplated by the reference to a particular chalcogenide moiety. In other embodiments, the chalcogenide compounds or moieties include two or more chalcogenide compounds.

### Polymers and matrices

A variety of polymers may be used in the embodiments disclosed herein. A polymer for such use may be biocompatible. It is to be understood that both non-biodegradable and/or biodegradable polymers may be used in the subject disclosure. As discussed below, the choice of polymer will depend in part on a variety of physical and chemical characteristics of such polymer and the use to which such polymer may be put.

Representative natural polymers and matrices include proteins, such as zein, modified zein, casein, gelatin, gluten, serum albumin, or collagen, and polysaccharides, such as cellulose, dextrans, hyaluronic acid, polymers of alginic acid, and natural fibrous matrix, such as filter paper.

Fibrous matrices contemplated by this disclosure include cellulose and cellulose based matrices, cellulose derivatives including cellulose acetate and cellulose phthalate, cellulose composite membranes, cellulose particles including micro- and nano-particles, fabrics such as linen, cotton, rayon, nylon and polyester based fabrics. Other matrices include silicon dioxide particles, such as fumed silica. In other embodiments, biocompatible matrices contemplated by this disclosure include silicon-containing polymers, hydrogels, etc.

Representative synthetic polymers include polyphosphazines, poly(vinyl alcohols), polyamides, polycarbonates, polyalkylenes, polyacrylamides, polyanhydrides, poly(phosphoesters), polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyphosphates, polyesters, and polyurethanes. For example, polymers may include polydimethylsiloxane, ethylene vinyl acetate, nylons, polyacrylics, polymethyl methacrylate, polyethylenes, polypropylenes, polystyrenes, poly(vinyl chloride) (PVC), and polytetrafluoroethylene (PTFE). Silicon rubbers may also be used as a polymer.

Synthetically modified natural polymers include alkyl celluloses, hydroxyalkyl celluloses, cellulose ethers, cellulose esters, and nitrocelluloses. Other like polymers of interest include, but are not limited to, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt.

In some embodiments, compositions of this disclosure include a biocompatible polymer. Examples of biocompatible polymers include poly(hydroxyvalerate), poly(L-lactic acid), polycaprolactone, poly(lactide-co-glycolide), poly(hydroxybutyrate), poly(hydroxybutyrate-co-valerate), polydioxanone, polyorthoesters, polyanhydrides, poly(glycolic acid), poly(D,L-lactic acid), poly(glycolic acid-co-trimethylene carbonate), polyphosphoesters, polyphosphoester urethanes, poly(amino acids), cyanoacrylates, poly(trimethylene carbonates), poly(iminocarbonate), copoly(ether-esters) (e.g. PEO/PLA), polyalkylene oxalates, polyphosphazenes and biomolecules such as fibrin, fibrinogen, cellulose, starch, collagen and hyaluronic acid. Polyurethanes, silicones, and polyesters may be used, as well as polyolefins, polyisobutylene and ethylene-alphaolefin copolymers; acrylic polymers and copolymers, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile; polyvinyl ketones; polyvinyl aromatics, such as polystyrene; polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins; polycarbonates; polyoxymethylenes; polyimides; polyethers; epoxy resins; rayon; rayon-triacetate; cellulose, cellulose acetate, cellulose butyrate; cellulose acetate butyrate; cellophane; cellulose nitrate; cellulose propionate; cellulose ethers; and carboxymethyl cellulose. Particular polymers contemplated herein include polyethyleneimine, polymethacrylate, polytetrafluoroethylene, and polydimethylsiloxane.

Polymers that resist protein adsorption may also be used in compositions contemplated by this disclosure. Such polymers include polyethylene glycols, polyurethanes and silicone elastomers, silica-containing polymers, and poly(vinyl)chlorides.

Other polymers that may be used include tecophilic polyurethanes, PDMS co-polymers, carbamates, and the like. In some embodiments, polymers that regulate water uptake may be used in embodiments of the disclosed composition. Polymers contemplated by this disclosure may also include those polymers that control the diffusion of selenium compounds, and/or polymers that control the diffusion of S-nitrosothiols.

All of the subject polymers may be provided as copolymers or terpolymers. These polymers may be obtained from chemical suppliers, or synthesized from monomers using standard techniques.

### Coatings and devices

Compositions and coatings contemplated herein include a matrix, such as one or more of the polymer(s) as described above, and a chalcogenide compound, such as an organoselenium, an organosulfur, and/or organotellurium compound; and/or an enzyme that includes selenium, sulfur and/or tellurium. In some embodiments, such a composition may produce nitric oxide when in contact, for example, with a bodily fluid such as blood.

Such compositions may be suitable for use as a layer or membrane, or in a layer or membrane, disposed, at least in part, on a sensing layer, or on an electrode surface of an analyte sensor, or on a medical device. In part, a biocompatible analyte-permeable composition of the present disclosure useful for use in analyte detection includes: (a) a selenium, tellurium, and/or sulfur compound; a selenium, tellurium, and/or sulfur containing enzyme; residues or moieties of the same; and/or combinations thereof, and (b) a biocompatible polymer that is at least partially permeable to the analyte(s) of interest.

In certain embodiments, a chalcogenide compound is incorporated into a polymer. The chalcogenide compound may be covalently attached to the polymer, dispersed throughout the polymer, and/or disposed on the surface of a polymeric layer or matrix. Various methods of covalent attachment of the chalcogenide compound or moiety may be employed. For example, innate amine groups (-NH₂) in selenocystamine may be reacted with a variety of reaction sites generally found in functionalized polymer backbones, such as carboxyl groups (-COOH), or aldehyde groups, (-CHO) and halides (-Cl, Br, I, F). Selenium and/or sulfur containing enzymes may be immobilized via a similar approach using any available reactive groups on the enzymes. In another embodiment, a reactive chalcogenide agent may be first coupled, bonded or associated with another small molecule, a protein, an enzyme, a polymer or a polymeric material to form a conjugate, and then further immobilized within or on the surface of a desired substrate, e.g., a metal surface of a medical device. In yet another embodiment, the catalytic moiety for S-nitrosothiol decomposition may be achieved through covalent coupling reactions of a functional group on the surface of a desired material such as polymeric films, glass surfaces, and/or the like.

In one alternative embodiment, hydrophobic organoselenium, organosulfur, or organotellurium compounds can be selected and incorporated into a material possessing a hydrophobic domain in its internal structure, for example, poly(vinyl chloride), polyurethanes, and the like. For such a composition, a chalcogenide compound may be added to a polymer or a composition including a polymer. A variety of methods are known in the art for encapsulating a substance in a polymer. For example, the agent or substance may be dissolved to form a homogeneous solution of reasonably constant concentration in the polymer composition, or it may be dispersed to form a suspension or dispersion within the polymer composition at a desired level of "loading" (grams of biologically active substance per grams of total composition including the agent, usually expressed as a percentage). For example, a composition comprising a chalcogenide compound may have 0.01%, 1%, 3% or even 5% or more by weight of a chalcogenide compound.

The terms "incorporated" and "encapsulated" are art-recognized when used in reference to an chalcogenide compound (or other material) and a polymeric composition, such as a composition of the present disclosure. The terms may contemplate any manner by which a chalcogenide compound or other material is incorporated into a polymer matrix, including, for example: attached to a monomer of such polymer (by covalent or other binding interaction) and having such monomer be part of the polymerization to give a polymeric formulation, distributed throughout the polymeric matrix, appended to the surface of the polymeric matrix (by covalent or other binding interactions), encapsulated inside the polymeric matrix, blending, mixing, swelling etc. The term "co-incorporation" or "co-encapsulation" refers to the incorporation of chalcogenide compound or other material and at least one other agent or other material in a subject composition. When a therapeutic agent is incorporated in, e.g., a matrix, it is to be understood that such therapeutic agent(s) can be released from such matrix in a contemplated fashion, e.g., the matrix may deliver a therapeutically effective amount of a therapeutic agent.

In an embodiment, the physical form in which any chalcogenide compound or other material is encapsulated in polymers may vary with the particular embodiment. For example, a selenium compound, tellurium compound, sulfur compound or other material may be first encapsulated in a microsphere and then combined with the polymer in such a way that at least a portion of the microsphere structure is maintained. Alternatively, a chalcogenide compound or other material may be sufficiently immiscible in the polymer, and as such, is dispersed as small droplets, rather than being dissolved, in the polymer. Any form of encapsulation or incorporation is contemplated by the present disclosure, in so much as the effectiveness over time of any encapsulated selenium compound or other material determines whether the form of encapsulation is sufficiently acceptable for any particular use. For example, the chalcogenide compound may be incorporated into a porous layer of the matrix or into pores that are part of a natural or synthetic matrix. In an embodiment, a polymer derivatized with a chalcogenide compound may then be cross-linked to form a hydrogel.

Suitable compositions may also include a wide range of additional materials. For example, materials may be incorporated into the compositions that alter the physical and chemical properties, including for example, the capability of preventing biofouling of the resulting composition and/or the analyte permeability of the composition. Without being limited thereto, such materials may include diluents, binders and adhesives, lubricants, disintegrants, colorants, bulking agents, flavorings, sweeteners, and miscellaneous materials such as buffers and adsorbents, in order to prepare a particular medicated composition. It is to be understood that such additional materials are selected so that none of these additional materials will substantially interfere with the intended purpose of the subject composition.

The subject compositions and coatings may include a pharmaceutically acceptable carrier. The term "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) glycols, such as propylene glycol; (5) polyols, such as glycerin, sorbitol, mannitol and polyethylene glycol; (6) esters, such as ethyl oleate and ethyl laurate; (7) buffering agents; (8) ethyl alcohol; (9) other non-toxic compatible substances employed in medical device coating formulations.

In addition to a chalcogenide compound, the subject compositions and coatings may contain therapeutic agents. Therapeutic agents in a subject composition may vary widely with the purpose for the composition. The term "therapeutic agent" includes without limitation, medicaments; vitamins; mineral supplements; substances used for the treatment, prevention, diagnosis, cure or mitigation of disease or illness; substances which affect the structure or function of the body; or pro-drugs, which become biologically active or more active after they have been placed in a predetermined physiological environment. It is to be understood that compositions contemplated by this disclosure may include one or more nitric oxide releasing or generating agents alone or in combination with one or more nitric oxide generating agents or chalcogenide compounds, and can include one or more other therapeutic agents.

Suitable "therapeutic agents" useful in the disclosure, include, but are not limited to, antithrombogenic agents (such as, for example, heparin, covalent heparin, hirudin, hirulog, coumadin, protamine, argatroban, D-phenylalanyl-L-poly-L-arginyl chloromethyl ketone, and the like); thrombolytic agents (such as, for example, urokinase, streptokinase, tissueplasminogen activators, and the like); fibrinolytic agents; vasospasm inhibitors; potassium channel activators (such as, for example, nicorandil, pinacidil, cromakalim, minoxidil, aprilkalim, loprazolam and the like); calcium channel blockers; antihypertensive agents; anti-infective agents including antiviral agents, antimicrobial agents and antifungal agents, antimicrobial agents or antibiotics (such as, for example, adriamycin, and the like); antiplatelet agents (such as, for example, aspirin, ticlopidine, a glycoprotein IIb/IIIa inhibitor, surface glycoprotein receptors and the like); antimitotic, antiproliferative agents or microtubule inhibitors (such as, for example, taxanes, colchicine, methotrexate, azathioprine, vincristine, vinblastine, cytochalasin, fluorouracil, adriamycin, mutamycin, tubercidin, epothilone A or B, discodermolide, and the like); antisecretory agents (such as, for example, retinoid, and the like); remodelling inhibitors; antisense nucleotides (such as, for example, deoxyribonucleic acid, and the like); anti-cancer agents (such as, for example, tamoxifen citrate, acivicin, bizelesin, daunorubicin, epirubicin, mitoxantrone, and the like); steroids (such as, for example, dexamethasone, dexamethasone sodium phosphate, dexamethasone acetate, β-estradiol, and the like); non-steroidal antiinflammatory agents (NSAID); COX-2 inhibitors; 5-lipoxygenase (5-LO) inhibitors; leukotriene A4 (LTA4) hydrolase inhibitors; 5-HT agonists; HMG-CoA inhibitors; antineoplastic agents, thromboxane inhibitors; decongestants; diuretics; sedating or non-sedating anti-histamines; inducible nitric oxide synthase inhibitors; opioids, analgesics; Helicobacter pylori inhibitors; proton pump inhibitors; isoprostane inhibitors; vasoactive agents; beta.-agonists; anticholinergic; mast cell stabilizers; immunosuppressive agents (such as, for example cyclosporin, rapamycin, everolimus, actinomycin D and the alike); growth factor antagonists or antibodies (such as, for example, trapidal (a PDGF antagonist), angiopeptin (a growth hormone antagonist), angiogenin, and the like); dopamine agonists (such as, for example, apomorphine, bromocriptine, testosterone, cocaine, strychnine, and the like); radiotherapeutic agents; heavy metals functioning as radiopaque agents (such as, for example, iodine-containing compounds, barium-containing compounds, gold, tantalum, platinum, tungsten, and the like); biologic agents (such as, for example, peptides, proteins, enzymes, extracellular matrix components, cellular components, and the like); angiotensin converting enzyme (ACE) inhibitors; angiotensin II receptor antagonists; renin inhibitiors; free radical scavengers, iron chelators or antioxidants (such as, for example, ascorbic acid, alpha tocopherol, superoxide dismutase, deferoxamine, 21-aminosteroid, and the like); sex hormones (such as, for example, estrogen, and the like); antipolymerases (such as, for example, AZT, and the like); antiviral agents; photodynamic therapy agents (such as, for example, 5-aminolevulinic acid, meta-tetrahydroxyphenylchlorin, hexadecafluoro zinc phthalocyanine, tetramethyl hematoporphyrin, rhodamine 123, and the like); antibody targeted therapy agents (such as, for example, IgG2 Kappa antibodies against Pseudomonas aeruginosa exotoxin A and reactive with A431 epidermoid carcinoma cells, monoclonal antibody against the noradrenergic enzyme dopamnine beta-hydroxylase conjugated to saporin, and the like); and gene therapy agents.

The compounds and compositions of the disclosure may also be administered in combination with other medications used for the treatment of diseases or disorders.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

In an embodiment, the composition may include lipophilic salts of nitrite/nitrate or nitrosothiols within its matrix to create a reservoir of nitrite/nitrate or nitrosothiol that, for example, can continuously leak to a surface.

Compositions of this disclosure may also include other agents that assist prevention of biofouling or microbial interference. Such agents include antifungals and antibiotics. For example, gentamycin and/or penicillin, and/or other broad-spectrum antibiotics and antifungals (e.g., ketaconazole) may be incorporated into the enzyme mixture or the polymer matrix to prevent microbial growth.

Plasticizers and stabilizing agents known in the art may be incorporated in polymers of the present disclosure. In certain embodiments, additives such as plasticizers and stabilizing agents are selected for their biocompatibility.

An embodiment of the composition of this disclosure may further contain one or more adjuvant substances, such as fillers, thickening agents or the like. In other embodiments, materials that serve as adjuvants may be associated with the polymer matrix. Such additional materials may affect the characteristics of the formed polymer matrix. For example, fillers, such as bovine serum albumin (BSA), mouse serum albumin (MSA), or silica particles, may be associated with or dispersed within the polymer matrix. For example, a filler may include a chalcogenide compound immobilized to a fumed silica particle. In certain embodiments, the amount of filler may range from about 0.1% to about 50% or more by weight of the polymer matrix. In other embodiments, the filler may be present in any of the following amounts: about 2.5%, 5%, 10%, 25%, or 40%. Other fillers known to those of skill in the art, such as carbohydrates, sugars, starches, saccharides, celluoses and polysaccharides, including mannitose and sucrose, may be used in certain embodiments in the present disclosure. Buffers, acids and bases may also be incorporated in the subject compositions to adjust the pH.

The charge, lipophilicity or hydrophilicity of any embodiment of the polymeric matrix may be modified by attaching or incorporating in some fashion an appropriate compound to the surface of a composition or membrane. For example, surfactants may be used to enhance wettability of poorly soluble or hydrophobic compositions. Examples of suitable surfactants include dextran, polysorbates and sodium lauryl sulfate. In general, surfactants are used in low concentrations, generally less than about 5%.

Binders are adhesive materials that may be incorporated in polymeric formulations to bind and maintain matrix integrity. Binders may be added as dry powder or as a solution. Sugars, natural polymers and synthetic polymers may act as binders. Materials added specifically as binders are generally included in the range of about 0.5%-15% w/w of the matrix formulation. Certain materials may exhibit multiple properties, such as microcrystalline cellulose, which is a spheronization enhancer, and may also have additional binding properties.

Various further coatings may be applied to modify the properties of a coating or composition. Three exemplary types of coatings are seal, gloss and enteric coatings. Other types of coatings having various dissolution or erosion properties may be used to further modify subject matrices behavior, and such coatings are readily known to one of ordinary skill in the art.

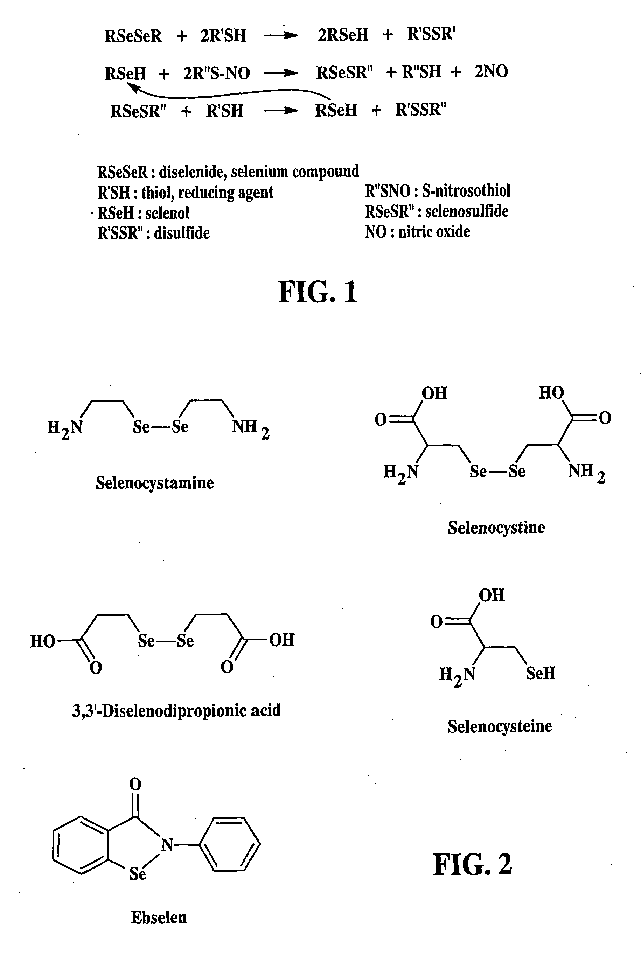

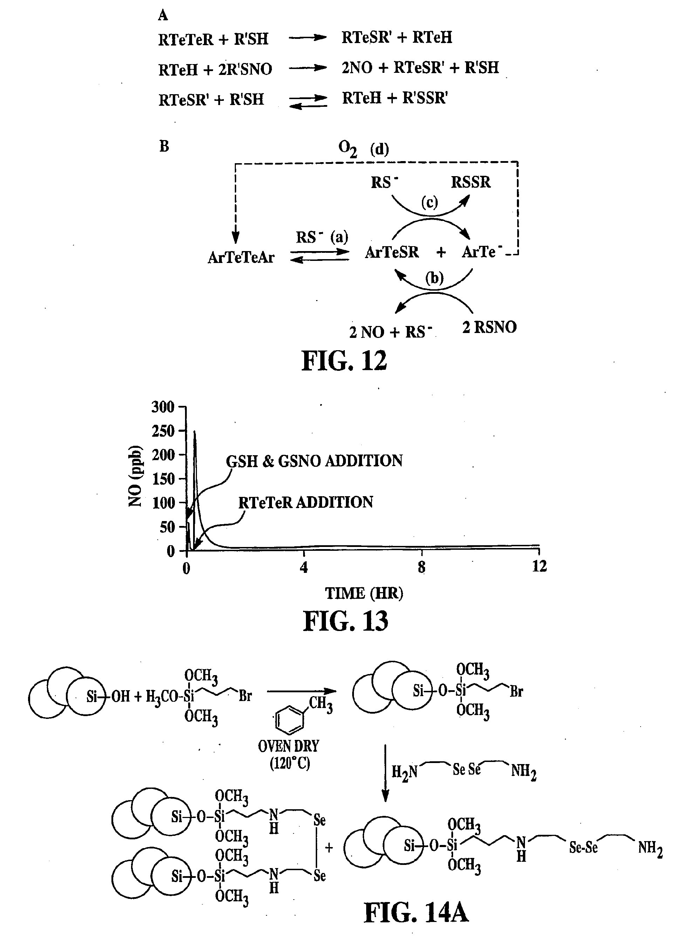

When a composition that includes a nitric oxide generating agent such as a chalcogenide compound, for example, an organoselenium, organothiol, or organotellurium compound and/or a selenium, tellurium or sulfur containing enzyme, is placed in contact with blood, for example, it may facilitate the conversion of endogenous S-nitrosothiols to NO as shown schematically in Figure 1 or Figure 12. During normal hemostasis, S-nitrosothiols in the blood may interact with a composition disclosed herein to produce NO at the surface of the polymer or polymer coating. In this manner, generation of NO locally from the surface of the polymer may prevent platelet adhesion. The concentration of endogenous S-nitrosothiols found in human blood include S-nitrosoalbumin, 0.25 - 7 µM; S-nitrosoglutathione, 0.02 - 0.2 µM; S-nitrosocysteine, 0.2 - 0.3 µM; S-nitrosohemaglobin, 0.3 - 0.003µM. For example, Figure 12 shows that a large amount of NO can be produced at the beginning of the reaction using organo-ditelluride and can continue to generate NO at a steady state. Such catalytic NO generation by, for example, diorgano telluride may generally occur in the presence of a reducing agent such as glutathione or cysteine.

Also contemplated by this disclosure are coatings for use on medical devices. Such a coating may include a polymer and a chalcogenide compound. A coating may include one or more layers, for example, a coating may include a first polymer layer including the chalcogenide compound, and optionally a second polymer layer. The first and/or second layer may further include one or more additional therapeutic agents. The second layer may be disposed on the first layer, or the first layer may be disposed on the second layer. The first and/or second layer may be biodegradable and/or hydrophilic.

As previously stated, compositions and coatings of the instant disclosure may be used, for example, on or in a medical device, and in some embodiments, on a metal surface of a medical device. "Medical device", as used herein, refers to any intravascular or extravascular medical devices, medical instruments, foreign bodies including implants and the like. Examples of intravascular medical devices and instruments include balloons or catheter tips adapted for insertion, prosthetic heart valves, sutures, surgical staples, synthetic vessel grafts, stents (e.g., Palmaz-Schatz, Wiktor, Crown, Mutlilink, GFX stents), stent grafts, vascular or non-vascular grafts, shunts, aneurysm fillers (including GDC, Guglilmi detachable coils), intraluminal paving systems, guide wires, embolic agents (for example, polymeric particles, spheres and liquid embolics), filters (for example, vena cava filters), drug pumps, arteriovenous shunts, artificial heart valves, artificial implants, foreign bodies introduced surgically into the blood vessels or at vascular or non-vascular sites, leads, pacemakers, implantable pulse generators, implantable cardiac defibrillators, cardioverter defibrillators, defibrillators, spinal stimulators, brain stimulators, sacral nerve stimulators, chemical sensors, breast implants, interventional cardiology devices, catheters, and the like. Examples of extravascular medical devices and instruments include plastic tubing, dialysis bags or membranes whose surfaces come in contact with the blood stream of a patient.

After a device or artificial material has been coated at least partially with a composition or coating as disclosed herein, it will be substantially suitable for its intended use, including, for example, implantation as a heart valve, insertion as a catheter, insertion as a stent, or for cardiopulmonary oxygenation or hemodialysis.

Also disclosed herein are methods for the administration of a therapeutically effective amount of the compounds and compositions described herein for treating cardiovascular diseases and disorders including, for example, restenosis, vasospasm, atherosclerosis, and diseases where vasodilation of arteries is indicated. For example, the patient can be administered a therapeutically effective amount of a composition contemplated herein. A therapeutically effective amount may be, for example, based on the amount of a chalcogenide compound necessary to provide a therapeutically effective amount of nitric oxide.

The term "therapeutic effect" is art-recognized and refers to a local or systemic effect in animals (particularly mammals, and more particularly humans) caused by a pharmacologically active substance. The term thus means any substance intended for use in the diagnosis, cure, mitigation, treatment or prevention of disease, or in the enhancement of desirable physical or mental development and/or conditions in an animal or human. The phrase "therapeutically-effective amount" means that amount of such a substance that produces some desired local or systemic effect, or for example, generates an amount of nitric oxide to produce some desired effect, at a reasonable benefit/risk ratio applicable to any treatment. The therapeutically effective amount of such a substance will vary depending upon the subject and disease condition being treated, the weight and age of the subject, the severity of the disease condition, the manner of administration and the like, which can readily be determined by one of ordinary skill in the art. For example, certain compositions of the present invention may be administered in a sufficient amount to produce a reasonable benefit/risk ratio applicable to such treatment.

Another embodiment of the disclosure provides methods for the inhibition of platelet aggregation and platelet adhesion caused by the exposure of blood (including blood components or blood products) to a medical device by incorporating a composition disclosed herein and disposing said medical device on or in a patient.

In some embodiments, a method is provided for patients in need thereof, for promoting angiogenic effects, such as enhancing vascularization/blood flow to ischemic cells/tissues. As a non-limiting example, the coatings or compositions may be used for promoting angiogenesis when coronary artery disease, e.g., ischemic myocardium, myocardial infarction, ischemic cardiomyopathy, or peripheral arterial disease, such as chronic limb ischemia claudication (skeletal muscle), rest pain/ischemic ulceration/gangrene is present or suspected. Treatment of a patient in need of promoting angiogenesis may be indicated in the event of, for example, ischemic stroke/neuropathy, such as brain/nerve tissue, for example, ischemic pneumbra around stroke/infarct.

A method is also provided to promote healing and/or endothelialization of intravascular luminal surfaces in a patient in need thereof, for example, to promote endothelialization of unstable/ulcerated atherosclerotic plaque, for example in coronary/carotid arteries, or on de-endothelialized luminal surfaces such as those found following an endarterectomy, for example within the carotid artery, thrombectomy (either/or arterial/venous), angioplasty, such as balloon, laser, or cryogenic angioplasty, an atherectomy, or following thrombolysis, by administering a composition disclosed herein.

Compositions provided herein may also assist in resolution of acute, or chronic arterial and/or venous thrombosis, for example revascularization and/or neovascularization and/or recanalization. In another embodiment, compositions are provided that promote development of neocapillary beds for gene therapy applications, organ regeneration applications, and for bioartificial hybrid organs (e.g. pancreas, kidney, lung, liver) placement. Methods are also provided to promote and/or enhance wound healing and/or for promoting granulation tissue, for example, for chronic wounds such as ischemic, diabetic, neuropathic, and venous statis based wounds.

In one embodiment, the compositions disclosed herein may be used to prevent fibrous tissue formation after incisions, or to treat neointimal hyperplasia. For example, a method is provided herein of treating a site of vascular compromise to seal a puncture or opening, and to treat, suppress or prevent a tissue response at such site, by administering a composition of this disclosure. In an embodiment, the method may include administering a nitric oxide agent or composition including a nitric oxide agent, such as the embodiments disclosed herein, or a nitric oxide agent and a hemostatic device or material, and applying, for example, the composition to the site.

Compositions disclosed herein may also be used to prevent incorporation and/or tissue encapsulation of medical devices, or surfaces thereof, for example, artificial or natural replacement surfaces, for example, placed in body cavities such as the thorax, abdomen, and/or hernia, devices such as implantable biosensors, for example intravascular, brain, heart, gut sensors, pacemakers/leads, implantable drug delivery systems, and other biomechanical devices/surfaces such as bioartificial organs, joints, or heart valves. The compositions disclosed herein may improve biocompatibility of, e.g., an implantable device such as a sensor, as compared to an implantable device that does not include a composition of the disclosure. For example, a device including an embodiment of the composition disclosed herein may be placed in the body, for example, for twice the duration as compared to a device without the disclosed composition, with substantially little or no adverse effect to the patient.

Another embodiment of the disclosure relates to local administration of a composition disclosed herein to the site of injured or damaged tissue (e.g., damaged blood vessels) for the treatment of the injured or damaged tissue. Such damage may result from the use of a medical device in an invasive procedure. Thus, for example, in treating blocked vasculature by, for example, angioplasty, damage may, in some instances, result to the blood vessel. Such damage may be treated by use of the compounds and compositions described herein. In addition to repair of the damaged tissue, such treatment may also be used to alleviate and/or delay re-occlusions, for example, restenosis. The compounds and compositions can be locally delivered using any of the methods known to one skilled in the art, including but not limited to, a drug delivery catheter, an infusion catheter, a drug delivery guidewire, an implantable medical device, and the like. In one embodiment, all or most of the damaged area is coated with a disclosed composition herein per se, or in a pharmaceutically acceptable carrier or excipient which serves as a coating matrix, including the matrix described herein. This coating matrix can be of a liquid, gel, semisolid or solid consistency. The composition can be applied in combination with one or more therapeutic agents, such as those listed herein. The carrier or matrix may be made of, or include agents which provide for metered or sustained release of the therapeutic agents.

In treating cardiovascular diseases and disorders, the compositions disclosed herein may be administered directly to the damaged vascular or non-vascular surface intravenously by using an intraarterial or intravenous catheter, suitable for delivery of the compositions to the desired location. For example, disclosed coatings, disposed on a medical device, may be used to deliver chalcogenide agents to desired location for generation of nitric oxide in-vivo. The location of damaged arterial surfaces is determined by conventional diagnostic methods, such as X-ray angiography, performed using routine and well-known methods available to one skilled in the art. In addition, administration of the composition using an intraarterial or intravenous catheter is performed using routine methods well known to one skilled in the art. Typically, the compound or composition is delivered to the site of angioplasty through the same catheter used for the primary procedure, usually introduced to the carotid or coronary artery at the time of angioplasty balloon inflation. The composition may slowly decompose at body temperature over a prolonged period-of time, releasing nitric oxide at a rate effective to treat cardiovascular diseases and disorders including, for example, restenosis.

"Cardiovascular disease or disorder" refers to any cardiovascular disease or disorder known in the art, including, but not limited to, restenosis, coronary artery disease, atherosclerosis, atherogenesis, cerebrovascular disease, angina, ischemic disease, congestive heart failure or pulmonary edema associated with acute myocardial infarction, thrombosis, high or elevated blood pressure in hypertension, vasospasm, platelet aggregation, platelet adhesion, smooth muscle cell proliferation, vascular or non-vascular complications associated with the use of medical devices, wounds associated with the use of medical devices, vascular or non-vascular wall damage, peripheral vascular disease, neoinitimal hyperplasia following percutaneous transluminal coronary angiograph, and the like. Complications associated with the use of medical devices may occur as a result of increased platelet deposition, activation, thrombus formation or consumption of platelets and coagulation proteins. Such complications, which are within the definition of "cardiovascular disease or disorder," include, for example, myocardial infarction, pulmonary thromboembolism, cerebral thromboembolism, thrombophlebitis, thrombocytopenia, bleeding disorders and/or any other complications which occur either directly or indirectly as a result of the foregoing disorders.

"Restenosis" is a cardiovascular disease or disorder that refers to the closure of a peripheral or coronary artery following trauma to the artery caused by an injury such as, for example, angioplasty, balloon dilation, atherectomy, laser ablation treatment or stent insertion. Restenosis may also occur following a number of invasive surgical techniques, such as, for example, transplant surgery, vein grafting, coronary artery bypass surgery, endarterectomy, heart transplantation, balloon angioplasty, atherectomy, laser ablation, endovascular stenting, and the like.

"Atherosclerosis" is a form of chronic vascular injury in which some of the normal vascular smooth muscle cells in the artery wall, which ordinarily control vascular tone regulating blood flow, change their nature and develop "cancer-like" behavior. These vascular smooth muscle cells become abnormally proliferative, secreting substances such as growth factors, tissue-degradation enzymes and other proteins, which enable them to invade and spread into the inner vessel lining, blocking blood flow and making that vessel abnormally susceptible to being completely blocked by local blood clotting, resulting in the death of the tissue served by that artery. "Blood" includes blood products, blood components and the like.

Also contemplated by this disclosure is a sensor for detecting analytes, such as for example, for detecting S-nitrosothiols in blood and/or tissue. Such analytes include nitrosothiol and glucose. Such sensors may be implantable for *in-vivo* use or subcutaneous use, or may be used externally on bodily fluids accessible without surgical or other invasive procedures. Alternatively, the disclosed sensors may be used on fluids analyzed remotely. Such sensors may be used to, for example, obtain measurements of the S-nitrosothiol content in a sample or patient over several days.

Embodiments of the coatings, compositions and methods disclosed herein may be used in combination with other treatment modalities in certain embodiments. As examples, the sensors, devices and methods of the present disclosure may be used in conjunction with surgery and/or with other sensors. Still further, the sensor disclosed herein may be capable of sensing more than one analyte simultaneously or in a step wise fashion, or may be used with systemic therapy, for example, insulin administration, or a combination of these modalities. For example, analyte sensors may be used in combination with a variable rate or programmable implantable insulin infusion pump.

Contemplated by this disclosure are analyte sensors, such as those in contact with bodily fluids of a patient, those in contact with an interstitial space in a patient, or those which contact blood subcutaneously or in a vein or artery, saliva, urine, perspiration, and the like. Electrodes for use in analyte sensors include those electrodes functioning on an amperometric basis.

The disclosure having been generally described, it will be more readily understood by reference to the following examples which are included merely for purposes of illustration of certain aspects and embodiments of the present disclosure, and are not intended to limit the disclosure in any way.

### EXAMPLES

### Example 1

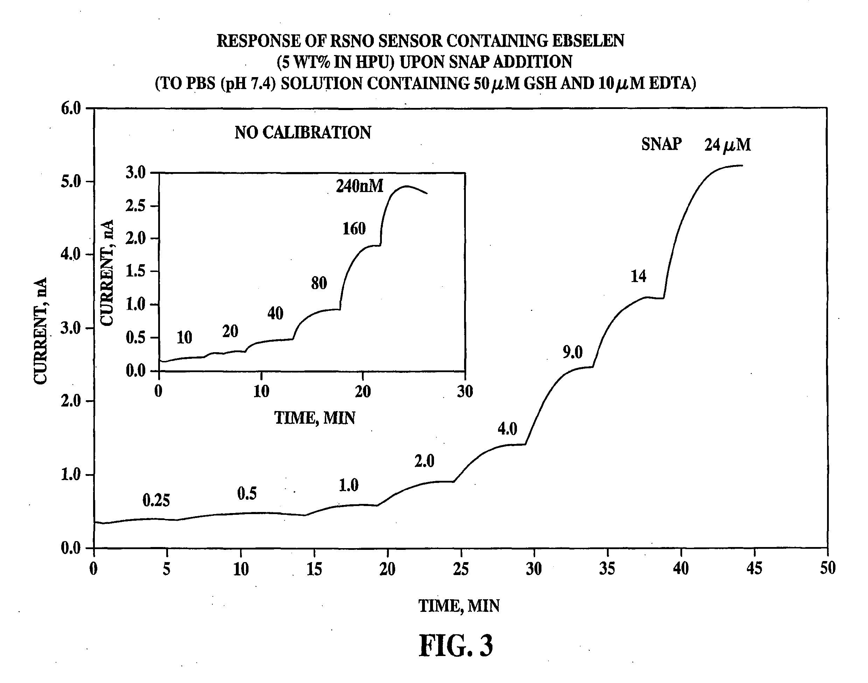

A hydrophobic organoselenium catalyst, ebselen, is incorporated into a hydrophilic polyurethane (Tecophilic, SP-60-A, 20%-water absorption) film that can be subsequently mounted on an electrochemical NO sensor for the detection of RSNO species. A NO sensor configuration is used that is similar to that disclosed in U.S. Patent Publication No. 2004/0224868_{;} hereby incorporated by reference in its entirety. 38 mg of the polyurethane is dissolved in 2 ml of THF solution containing 2 mg of ebselen. The polymeric film doped with catalytic selenium agent is prepared by casting 0.5 ml of the cocktail solution onto a 2.4 cm² glass slide. A patch of the resulting film is added as an outer catalytic membrane at the distal tip of an amperometric NO selective sensor to create an RSNO sensor. Figure 3 shows the quantitative amperometric responses of the resulting RSNO sensor at various given concentrations of S-nitroso-Nacetyl-DL-penicillamine (SNAP) between 0.5- 25 µm. Example 2: Surface Immobilization

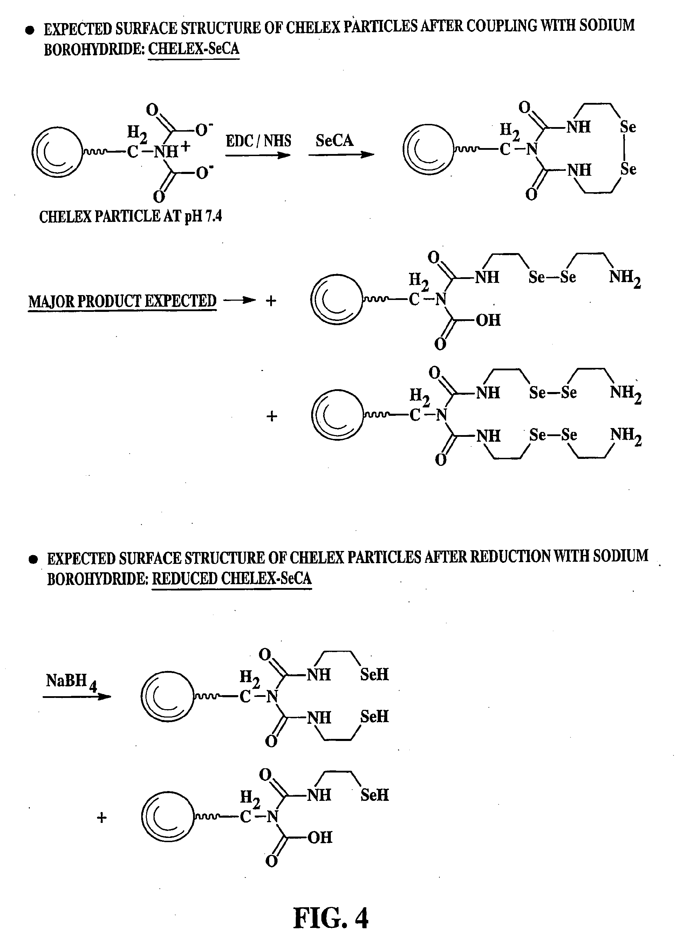

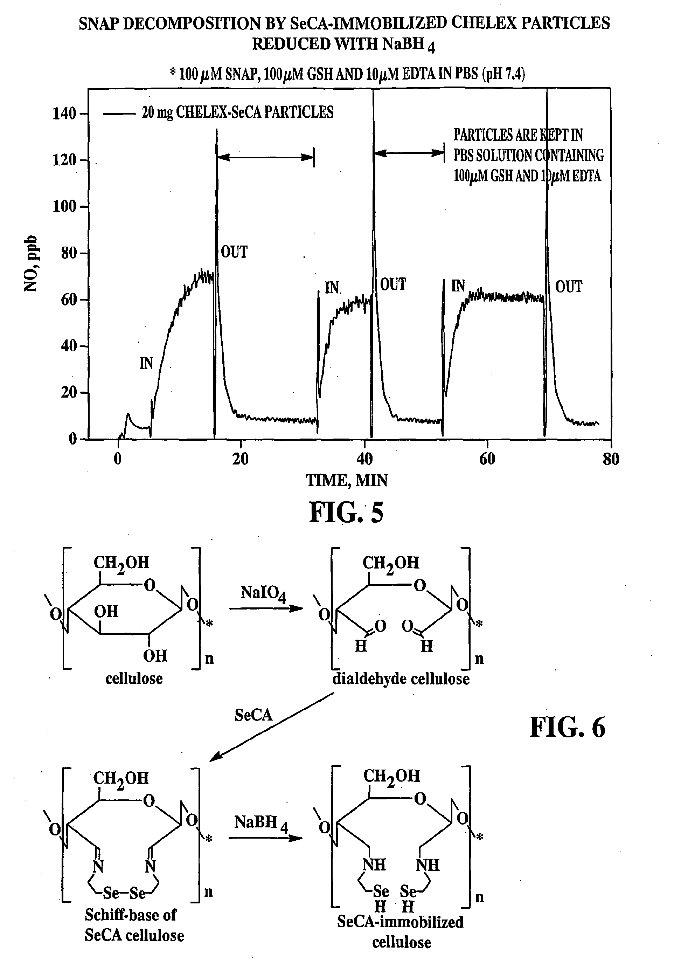

Surface carboxyl groups on metal-chelating resin particles (Chelex, 0.6mmol-carboxyl group/g, ∼0.5mm particle size) are coupled with the amine ends of selenocystamine. 1 g of Chelex resin particles dispersed in 10 ml of MES buffer (25 mM, pH 6.7) is incubated for 10 min with 1-(3-dimethylaminopropyl)-3-ethylcarbodiimide (EDC or EDAC, 400mg) and N-hydroxysuccinimide (NHS, 70 mg) to activate the carboxyl groups. Then, a selenocystamine (SeCA) solution (SeCA 200 mg in 5 ml MES buffer) is added to the incubated reaction mixture and is subsequently allowed to react for 40 min. Yellowish particles are obtained and further reduced in 20 ml of 0.1M NaBH4 solution to cleave the un-reacted half of the immobilized SeCA species and create selenol groups on the surface of the particles (see Figure 4 for a schematic of a reaction scheme). 10 mg of the resultant clear (no color) particles are encapsulated in a thin nylon mesh (∼50µM pore size) and are found capable of catalyzing S-nitroso-N-acetylpenicilamine (SNAP) decomposition as observed with chemiluminescence monitoring of the generated NO (see Figure 5).

### Example 3: Covalently attached organo-selenium species to a polymer backbone

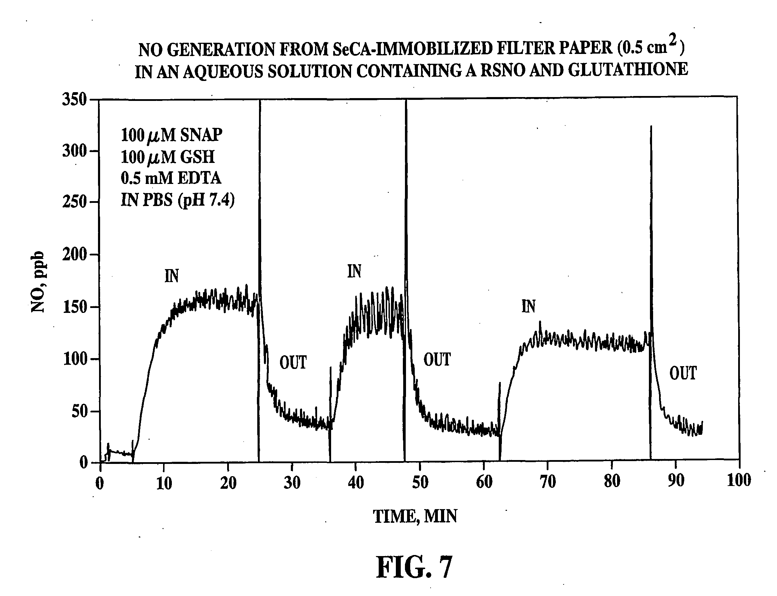

Selenocystamine (SeCA) is immobilized onto a filter paper by reacting the SeCA with dialdehydes created by diol-group oxidation on beta glucose unit as shown in Figure 6. First, the surface of the filter paper (Whatman 50, 55-mm diameter) is oxidized in 0.1M NaIO₄ solution for 3 hours, and then reacted with SeCA (50 mM in 0.1 M Tris buffer, pH 8.2) for 1 hour. The resultant Schiff-base linkages and diselenide bonds are further reduced to form C-N single bond and selenol groups in 0.1 M NaBH₄ for 1 hour. The obtained paper is stored in 0.1 M phosphate buffer (pH 4.5) solution overnight and washed before use. The reduction reaction is quenched with 0.1 M HCl solution. A patch of the resultant modified filter paper is found to decompose RSNO catalytically from the results of NO flux changes in the presence/absence of the modified filter paper in RSNO solution as shown in Figure 7.

### Example 4: Long term NO generation by organo-selenium-immobilized polymer

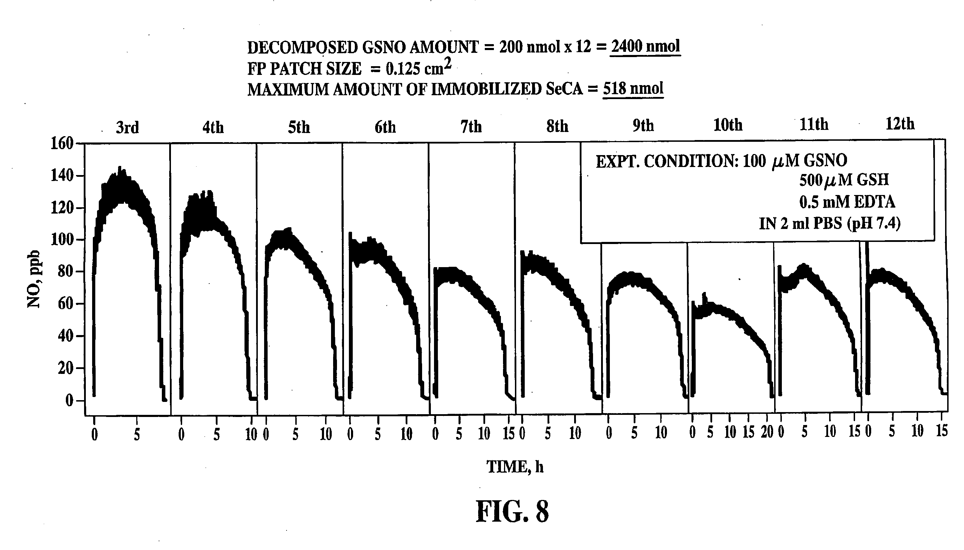

A small patch (area, 0.125 cm²) of Se-immobilized filter paper (Se-FP) prepared as described in Example 3 can be used for monitoring long term NO generation from a PBS (pH 7.4) solution containing 100 µM GSNO, 500 µM GSH and 0.5 mM EDTA. NO generation/production is measured via chemiluminescence detection. The entire given amount of GSNO is decomposed, NO production ceased in each batch of tests, and the reactions are resumed in a fresh buffer solution containing all components mentioned above (GSNO, GSH AND EDTA) with the same initial concentrations. As shown in Figure 8, a patch of Se-FP can consume all the given amount of RSNO to produce NO up to 12 batches of tests, where a total of 2.4 µmol (0.2 µmol x 12) of GSNO is decomposed. The Se-loading on a filter paper is separately estimated at less than ∼ 4.1 µmol/cm² from the UV/VIS absorbance change of SeCA (λₘₐₓ = 300 nm) solution during the Se-immobilization procedure described in Example 3. Based on this calculation, it is clear that a Se-FP patch can consume GSNO continuously and decompose more GSNO (2.4 µmol) than the immobilized Se amount in the given Se-FP patch (∼0.52 µmol in 0.125 cm² of Se-FP).

### Example 5: S-nitrosothiol detection by using NO sensor modified with organo-selenium-immobilized polymer

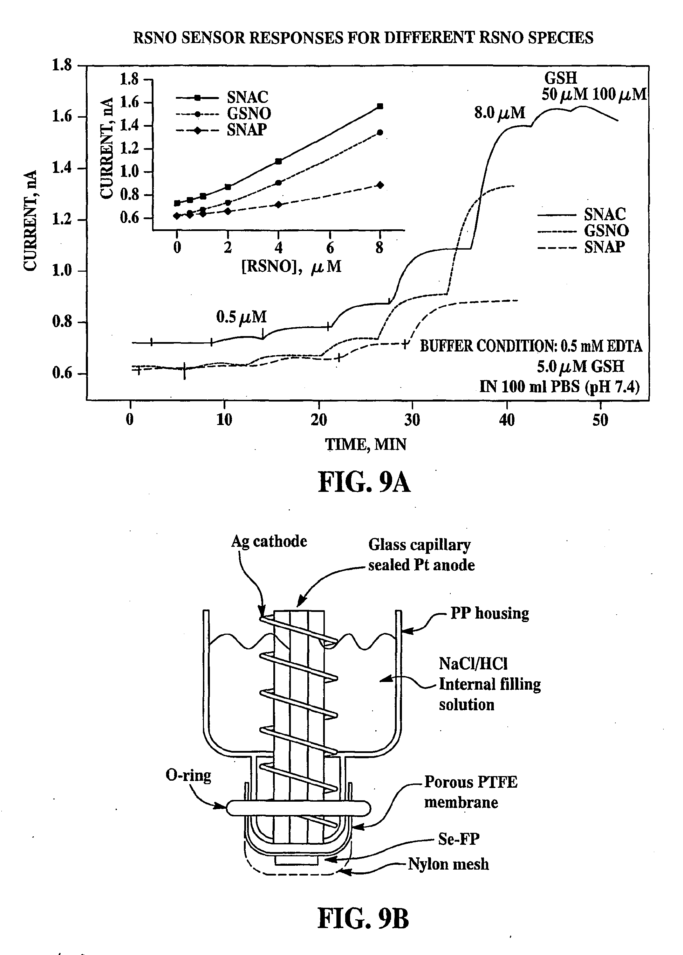

A small patch of Se-immobilized filter paper (Se-FP) prepared as described in Example 3 is mounted on an amperometric NO sensor to detect RSNO species in solution phase as shown in Figure 9B. Such modified NO sensors can produce amperometric current signals due to the production of NO by the catalytic RSNO decomposition on Se-FP and the subsequent oxidation of NO on the platinum electrode. Figure 9A shows that the sensor's amperometric response patterns are reproducible and quantitative upon the concentration changes (0.5 µM ∼ 8 µM) of RSNO species in the given PBS (pH 7.4) solution containing 0.5 mM EDTA and 5 µM GSH. The sensitivity of such RSNO sensors may be dependent on the nature of the RSNO species; Figure 9A (inset, current vs. concentration curves) shows GSNO produces larger current level changes than SNAP.

### Example 6: Control experiment on the amperometric RSNO detection with the modified NO sensor

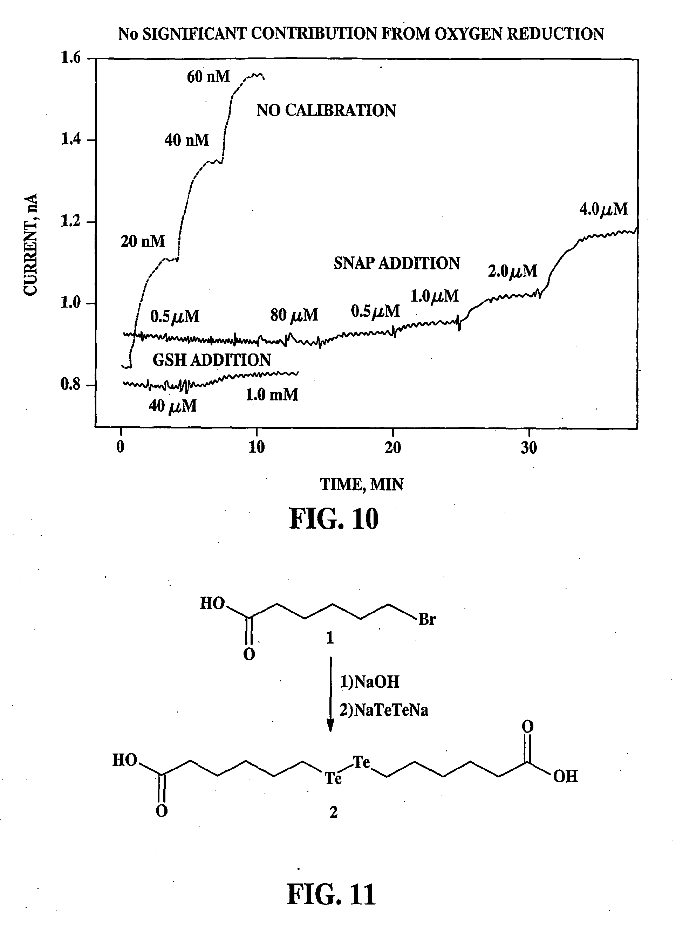

In ambient conditions, Se-compounds including selenocystamine have been shown to reduce oxygen at the expense of GSH with production of H₂O₂, as an intermediate of oxygen reduction. Since, however, H₂O₂ is a potential interfering molecule for the given configuration of NO sensor, the modified NO sensor's performance is tested by increasing GSH concentration in the absence of GSNO. Figure 10 demonstrates that negligible current changes are found even upon significant GSH concentration increases, which implies no significant contribution from oxygen reduction by Se-FP on the modified NO sensor during the measurement, and that the current level changes obtained in representative previous Examples appear to be due to the presence of NO created by catalytic RSNO decomposition on Se-FP surface.

### Example 7: Preparation of diorgano ditelluride

Compound 2 (see Figure 11) is prepared *in situ* by using disodium ditelluride solution and 6-bromohexanoic acid sodium salt in water at 80°C, following the reaction schematic in Figure 11.

### Example 8

Figure 13 shows the catalytic activity of diorgano ditelluride for decomposing S-nitrosoglutathione (RSNO) or S-nitrosocysteine (CySNO) to NO in the presence of a reducing agent such as glutathione (GSH) at the physiological pH. Diorgano ditelluride, (compound 2) is able to generate much more NO than the amount of diorgano ditelluride, 2 used from GSNO and GSH in PBS buffer (pH 7.4) in the presence of EDTA at the ambient temperature.

### Example 9: Derivatization of fumed silica

Selenium groups may be anchored onto the fumed silica surface using amine-containing silylating reagents (e.g., 3-bromopropyl rimethoxy silane) via a two step synthesis as shown in Figure 14A. Fumed silica particles are reacted with 3-bromopropyl trimethoxy silane to provide a linking site for selenocystamine. The silylated particles are then reacted with selenocystamine to introduce selenium sites onto the fumed silica particles. These reactions are carried out and the derivatized particles are collected for analysis.

### Example 10

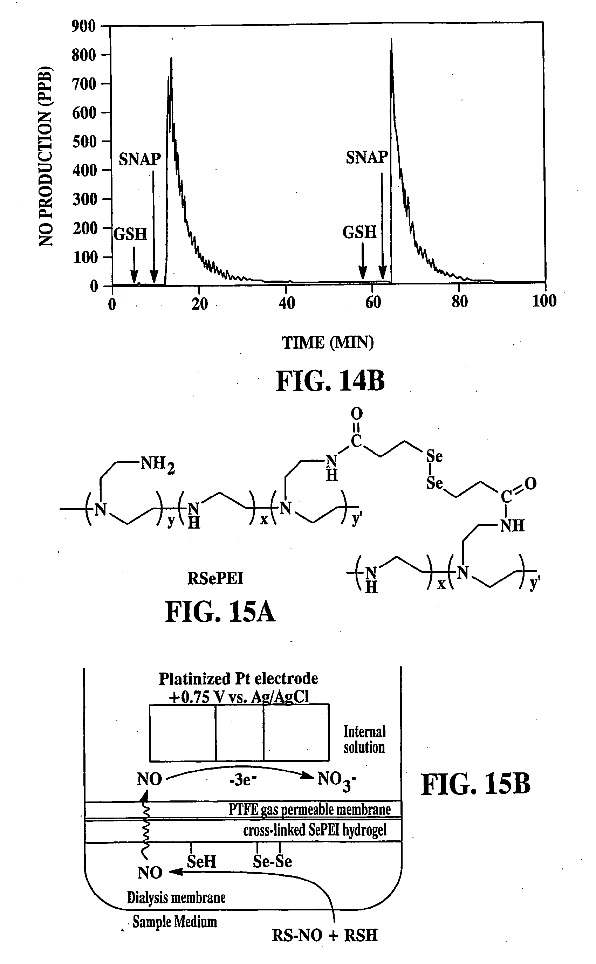

Nitric oxide production is measured from the particles of Example 9 via chemiluminescence, a direct measurement technique for NO, by soaking the particles in PBS buffer at 37 °C and then adding aliquots of S-rutrosothiol at interval time points. As shown in Figure 14B, the derivatized fumed silica particles cause NO to be generated from the S-nitrosothiol until the S-nitrosothiol is consumed, at which point additional aliquots of S-nitrosothiols are added and the process is repeated several times. This process may be carried out continuously, as long as a source of S-nitrosothiols is present. The human body contains a steady-state concentration of S-nitrosothiols in the range of 0.2 µM to 7 µM; thus the ability to continuously generate NO from the catheter surface is substantially endless. Addition of S-nitrosothiol to buffer alone or fumed silica alone does not produce NO, thus the NO produced from the derivatized particles are a result of the selenium modification.

### Example 11: Derivatization of PEI with SeDPA

A diselenide, 3, 3' dipropionicdiselenide (SeDPA) is covalently attached to polyethyleneimine (PEI) to create the selenium containing PEI (RSePEI; see Figure 14) by using an EDC/NHS coupling method. The carboxylic acid groups of SeDPA are first activated by EDC and NHS to form N-succinimide esters. A solution of PEI (40 mg/mL; either 25k or 750k avg. MW)) in 2- [N-morpholino]ethanesulfonic acid sodium salt (MES, pH 5.8) buffer is reacted with the activated SeDPA (12.5 mM), EDC and NHS at room temperature for 2 hours. The molar ratio of EDC:NHS:RSe-COOH is adjusted to 6:4:1 to achieve the maximum coupling of diselenides to the PEI. The resulting RSePEI solution is dialyzed (MWCO, 15kD), first against the same MES buffer and then DI water for 1 day. For RSePEI derivatized with SeDPA, the non-covalently linked SeDPA species are removed by reducing the diselenide bond with sodium borohydride (20 mM), and then the reaction mixture is exhaustively dialyzed against 50 mM NaCl first, and then DI water for 3 days. Control PEI is also prepared in the same manner, except without the addition of the diselenide to the coupling reaction. The RSePEI material obtained is used as a fresh solution or is stored after lyophilization. The dry polymers are found to contain 3.6 ± 0.3 and 3.9 ± 0.3 w/w% (for avg. Mw 25k and 750k PEI, respectively) of Se by ICP-MS analysis. Based on the assumption that the Se-content after the coupling reaction represents the actual coupling efficiency solely with primary amine groups, a small fraction (∼6 %) of the total primary amine groups in PEI is consumed and the remaining free amines are available for further immobilization onto filter paper or dialysis membranes. Example 12: Cross-linked SePEI Hydrogel Formation on Dialysis Membrane (DM)

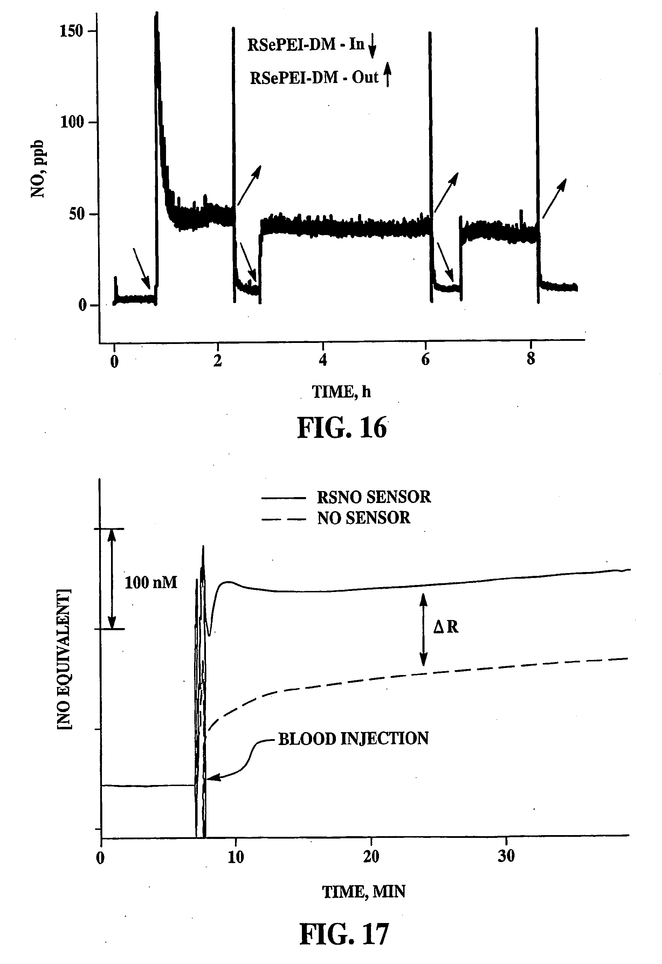

A tube of DM (MWCO, 25kD) containing 10 mL of 1 wt% RSePEI (derivatized from avg. Mw 25k PEI) and 0.1 mM EDTA in 0.1 M MOPS buffer (pH 7.9) solution, is first soaked in a solution of this same composition for at least 2 days. After washing with MOPS buffer, the tube of DM is soaked in a glutaraldehyde solution (1 wt%) for 20 min to crosslink the RSePEI species within pore structure of the dialysis membrane. The resulting DM tubing is washed with DI water and subsequently soaked in 10 mM sodium borohydride solution to reduce imine bonds for 1 hour. All reactions are carried out with gentle shaking or stirring. After discharging all solution, the inside/outside of the modified DM (RSePEI-DM) tubing is washed again and stored in 0.1 M phosphate buffer (pH 4.3) until use. Control pieces of DM are prepared with control PEI (no RSe attached), in a similar manner. Small pieces of the tubing walls are cut as needed, and tested for NO generation. Figure 16 depicts the solution phase like surface catalytic activity by generating NO in two discrete modes F (fast) and S (slow). Example 13: Fabrication of Amperometric NO/RSNO Sensors

A platinized Pt working electrode (Pt disk (with 250-µm o.d.)) sealed in glass wall tubing and a Ag/AgCl wire as the reference/counter electrode are employed in a gas sensing configuration. The two electrodes are incorporated behind a PTFE gas-permeable membrane (GPM). To create the RSNO sensor to demonstrate NO generation from RSNOs in fresh blood, a piece of RSePEI-DM is affixed on the GPM of the NO sensor using an O-ring, allowing the RSePEI-treated surface to face toward the GPM. Control NO sensors are also prepared with control DM described above (no RSe species). All sensor polarization, calibration and subsequent amperometric measurements are carried out in a standard fashion. Example 14: Detection of RSNOs in Blood

Animal blood is freshly obtained from Extracorporeal Membrane Oxygenation (ECMO) laboratory at the University of Michigan Medical School. Blood samples are heparinized immediately after being obtained by using a concentrated heparin solution (2 U/ml) added at 1:500 volume ratio to the blood (from porcine intestine, Sigma-Aldrich (St. Louis, MO)). The resulting blood samples have ACT (activated clotting time) values in the range of 250 ∼ 300 seconds, and are kept at 25 °C in the dark and used within 3 hours. To investigate whether exposure to blood plasma affects the NO generating ability of the RSePEI-FP material, platelet-rich plasma is prepared from heparinized porcine blood via centrifugation at 250 g for 15 min. A piece (0.5 cm²) of the RSePEI-FP polymer is stored in the plasma for up to 5 days, and its catalytic NO generation from RSNO species is tested intermittently by NOA measurements after simple washing with DI water. For direct detection of NO generated in rabbit blood using the immobilized RSePEI species, two electrodes, a control NO sensor and a RSNO sensor are employed. Each sensor is first calibrated with respect to its inherent response to NO in PBS buffer. Then, the amperometric signals of both sensors are stabilized at 25°C with the sensors placed into the same N₂-saturated 60 ml PBS (pH 7.4) solution. Finally, 40 ml of the fresh whole blood sample is added to the PBS solution to yield a 40% (v/v) dilution under a N₂ atmosphere, and the amperometric responses of each sensor to the sample is monitored.

As illustrated in Figure 17, the RSNO sensor exhibits a greater increase in amperometric NO response upon injection of rabbit blood into PBS (pH 7.4) at 25 °C, compared to that exhibited by the control NO sensor. The difference in NO levels detected by the two sensors, i.e., ΔR, strongly suggests the degradation of endogenous RSNOs predominantly occurs when the immobilized RSe catalyst is present. The catalytic layer yields an increase in response equivalent to a change of approximately 85 nM (ΔR) in effective NO levels localized at the surface of the device. Both sensors are pre-calibrated for their direct response to NO. Also, due to the MWCO (25 kDa) of the dialysis membrane used, LMW-RSNOs are likely to be converted to NO at the surface of the sensor, not S-nitrosoproteins such as AlbSNO. The response patterns shown in Figure 17 are reproducible, and have been observed in several separate experiments using fresh rabbit blood.

### Example 15: Synthesis of 5,5'-ditelluro-2,2'-dithiophenecarboxylic acid (DTDTCA, 2)

To a stirred solution of 2-thiophencarboxylic acid (2.0 g, 15.6 mmol) in THF (200mL) is added NaH (95 %, 0.66 g, 15.7 mmol) at 0 °C. After 10 minutes, n-BuLi (2.5 M solution in hexanes, 6.3 mL, 15.7 mmol) is slowly dropped into the above solution and stirred for 10 minutes. The reaction mixture is warmed to RT, then, stirred for 50 minutes. Tellurium (1.9 g, 14.9 mmol) is quickly added into the reaction mixture under a strong stream of nitrogen. After stirring for 2 hours, the mixture is concentrated under a reduced pressure to yield about 20 ml of a reddish brown slurry. The slurry is poured into a solution of DI water (300 mL) and CH₂Cl₂ (200 mL) at 0 °C while adjusting the pH of solution to approx. 1 using 1.5 N HCl. The entire mixture is vigorously mixed by blowing air through it at 0 °C. The mixture is filtered to remove an undissolved solid and the filter cake is washed with CH₂Cl₂. The separated water layer is extracted 2 times more with CH₂Cl₂. The combined organic layer is dried with anhydrous Na₂SO₄, filtered and washed with CH₂Cl₂. The filtrate is concentrated to give a dark reddish solid under a reduced pressure.

The crude residue is triturated with CH₂Cl₂ (50 mL), then filtered and washed with CH₂Cl₂ to give a reddish brown solid (0.93 g, 25 % yield). Decomposition Temperature 168 - 172 °C; ₁H NMR (500 MHz, DMSO-d₆, 25 °C): δ=13.17 (bs, 2H; 2 COOH), 7.54 (d, J= 4.5 Hz, 2H; 2 CCH), 7.43 (d, J= 4.5 Hz, 2H; 2 TeCCH); ₁₃C NMR (12S MHz, DMSO-d₆, 25 °C): δ=162.28, 142.13, 140.12, 134.53, 106.19.; ₁₂₅Te NMR (MHz, DMSO-d₆, 25°C): δ=497.60; IR (KBr)= 3426 cm⁻¹ (COO-H), 2959, 2554 cm⁻¹ (=C-H), 1667 cm₋₁ (C=O), 1516 cm₋₁ (C=C), 1422 cm⁻¹ (=C-H); HRMS(EI): *m*/*z*: [M]+ calcd. for CtoH60₄S₂Te₂, 513.7832: 513.7835.; Anal. Calcd for C₁₀H₆O₄S₂Te₂; C, 23.57; H, 1.19; O, 12.56; S, 12.59: Found C, 23.25; H, 1.23; S, 12.28.

### Example 16: Tellurosulfide polymer

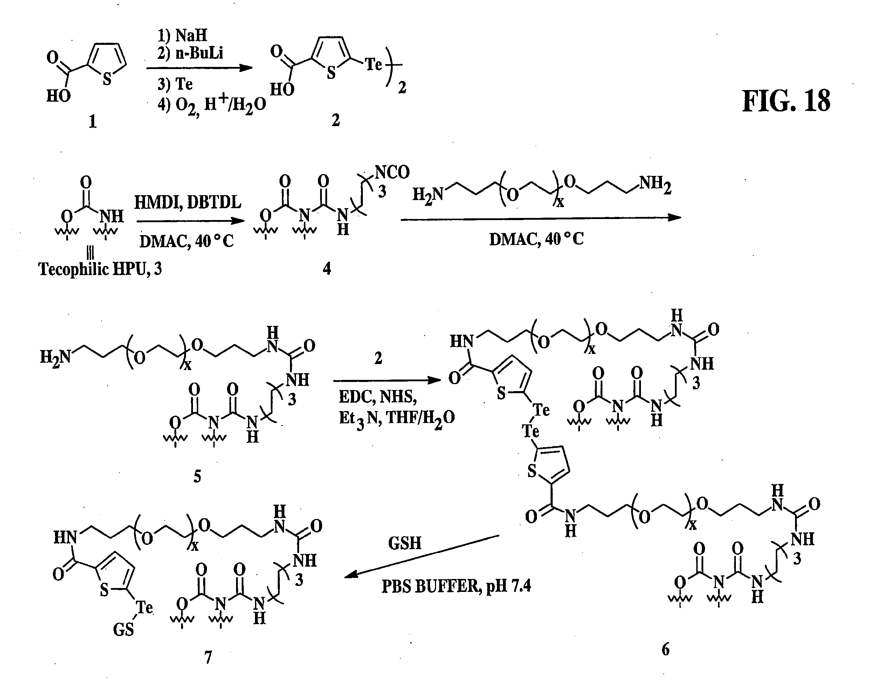

A synthetic route to tellurosulfide polymer 7 is depicted in Figure 18. Hydrophilic polyurethane (Tecophilic, SP-93A-100) is purified by soxhlet extraction prior to use. A dried HPU (2.0 g, ca 4.8 mmole of urethane group) is dissolved in anhydrous DMAC (40 mL). This solution is dropwise added into a stirred solution of HMDI (3.89 ml, 24 mmole) and DBTDL (72 µL, 0.12 mmole) in DMAC (4 mL) at 40 - 45°C. for 3 hours. After 1.5 days, the mixture is cooled down to RT and then is slowly added into anhydrous Et₂O (400 mL). The solid formed is filtered and washed with anhydrous Et₂O (600 mL). The filter cake is dried with N₂ blowing followed by vacuum drying to afford a white polymer, the desired product (2.0 g). IR (film on NaCl)= 3323 cm₋₁ (N-H), 2927,2858 cm₋₁ (CH₂), 2264 cm₋₁ (NCO), 1715 cm₋₁ (C=O), 1615 cm₋₁ (HNCONH), 1528 cm₋₁ (C-N, N-H), 1101 cm₋₁ (CH₂-OCH₂.

### Aminated Polymer 5:

Polymer **4** (1.86 g) is dissolved in anhydrous DMAC (30 mL), and then is slowly added into a stirred solution of dipropylamine-PEO (10.4 g) in DMAC (12 mL) at 40 °C for 3 hours. The mixture is stirred for 1 day at 40°C, and then is slowly added into Et₂O (400 mL). The yellowish polymer formed is filtered and washed with Et₂O (600 mL). The filter cake is soxhlet extracted with MeOH for 2 days. After cooling to RT, the solid cake is again washed with MeOH, then dried by vacuum pump for 2 days to yield polymer **5** (0.82 g). A solution of aminated polymer **5** in DMAC is titrated by a calorimetric method using bromophenol blue and p-toluenesulfonic acid in isopropanol (0.2 mmole of amine sites/g of polymer **5**). IR (film on NaCl)= 3323 cm₋₁ (N-H), 2916, 2857 cm₋₁ (CH₂), 1715 cm₋₁ (C=O), 1614 cm₋₁ (HNCONH), 1529 cm₋₁ (C-N, N-H), 1102 cm₋₁ (CH₂-O-CH₂)

### Ditelluride Polymer 6:

DTDTCA 2 (17 mg, 33 µmole) solution in THF (5 mL) is mixed with EDC.HCl (15 mg, 78 µmole) in DI water (5 mL). The cloudy mixture is stirred and became clear by adding Et₃N (20 mg, 198 µmole). Then, NHS (9 mg, 78 µmole) is added into the mixture at RT. Aminated polymer **5** (0.34 g, 68 µmole of free amine) solution in THF (12 mL) is then mixed with the above solution and stirred at RT overnight. The mixture is slowly added into Et₂O (900 mL) to form a slightly reddish yellow polymer. The solid is washed with Et₂O and DI water. The filter cake is stirred in MeOH at RT overnight. The residue is again filtered and washed with MeOH, and then is dried with a vacuum pump to give a yellowish polymer 6 (0.2 g). IR (film on NaCl)= 3320 cm⁻¹ (N-H), 2915, 2849 cm⁻¹ (CH₂), 1715 cm⁻¹ (C=O), 1616 cm⁻¹ (HNCONH), 1526 cm⁻¹ (C-N, N-H), 1445 cm⁻¹ (=C-H), 1099 cm⁻¹ (CH₂-O-CH₂).

### Tellurosulfide Polymer 7:

A small film of polymer **6** (3.92 mg; size, 0.9 cm X 1.8 cm; thickness, 2.4 µm) is soaked in the solution of GSH/GSNO (glutathione/s-nitrosoglutathione) (100 µM/100 µM) in 10 mL of PBS buffer (lOmM), pH 7.4 containing 0.5 mM EDTA (the same PBS buffer used unless otherwise noted). After the mixture is shaken overnight at RT, the film is taken out from the mixture. This film is again shaken in a fresh solution of GSH/GSNO (200µM/200 µM) in 10 mL of PBS buffer for 6 hours. The film is removed from the solution and then put into a fresh solution of 10 mL PBS buffer. The same procedure to wash the film is operated a couple of times to give a film of desired polymer **7** as a hydrated state right before using in NOA experiments. IR (film on NaCl)= 3326 cm-1 (N-H), 2923, 2859 cm-1 (CH₂), 1716 cm⁻¹ (C=O), 1662 cm-1 (C=O from GSH), 1615 cm-1 (HNCONH), 1531 cm-1 (C-N, N-H), 1450 cm-1(=C-H), 1249 cm-1 (CH₂ from GSH), 1108 cm-1 (CH₂-O-CH₂).

### Example 17

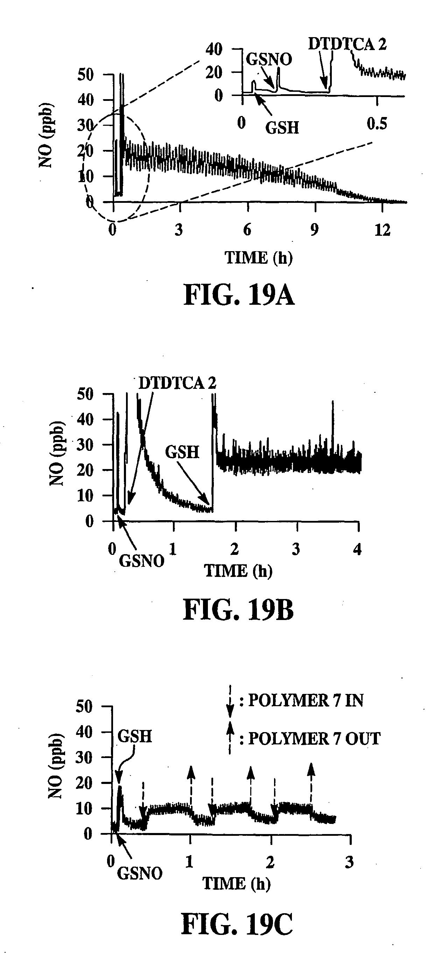

As shown in Figure 19A, a relatively large signal appears quickly upon adding catalytic amounts of DTATCA **2**. Figure 19A also depicts other measurements of catalytic NO generation by ditelluride compounds. (2.5 µM) into a solution containing GSNO (25 µM) and GSH (100 µM) in PBS buffer. The rate of NO generation slowly decreases with time to reach a steady-state that lasts until all of the GSNO is consumed. DTDTCA 2 decomposes RSNO to NO even in the absence of RSH, see Figure 19B. Polymer 7 exhibits catalytic NO generation from a solution of GSH/GSNO in PBS buffer as shown in Figure 19C.

### Example 18: Organotelluride tethered poly(allylamine hydrochloride) crosslinked with a poly(2-hydroxyl ethyl methacrylate (pHEMA) and poly(ehtylen glycol methacrylate (PEGMA) hydrogel

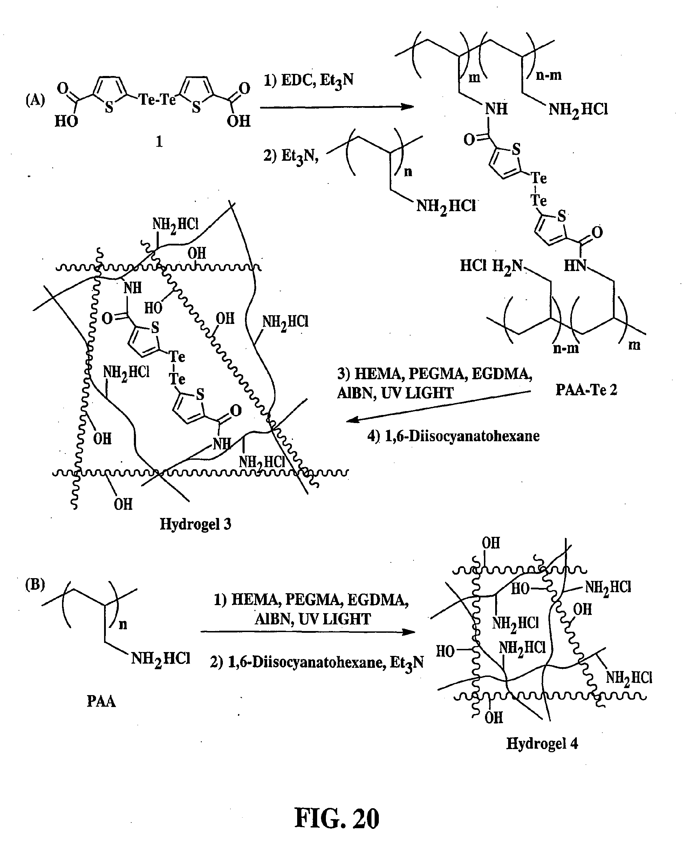

Synthesis of PAA-Te 2 (see Scheme 1 (A), Figure 20). DTDTCA **1** (40 mg, 0.079 mmol) and Et3N (35 mg, 0.35 mmol) are dissolved in THF (2 ml, distilled prior to use). After adding a solution of EDC.HCl (0.16 mmol) in DI water (0.2 ml) into the above solution, the mixture is stirred for 30 minutes at RT. Then, a solution of PAA hydrochloride (Mw 70,000, 300 mg, 4.3 mmol) and Et3N (35 mg, 0.35 mmol) in DI water (2 ml) is poured into the mixture and stirred for 1 day. After removing THF by evaporation, the reaction mixture is filtered with a membrane filter (Mw cutoff, 30,000) via centrifugation, and washed with brine several times to remove water soluble small molecules. The residual is dried by lyophylization. The resulting solid is stirred in THF for 5 hours, and is then filtered and washed with THF. The filter cake is dried by nitrogen flow to yield a reddish brown colored polymer solid of PAA-Te **2**. 1H NMR analysis indicates that 1.3 mol % amine groups of the polymer backbone are coupled with DTDTCA (theoretical value, 1.8 mol %). Synthesis of hydrogel 3, (see Scheme 1 (Figure 20A)) 2-hydroxyethyl methacrylate (HEMA purified by distillation before using, 210 mg, 71.9 wt %), PEGMA, (Ave. Mw 526, 54 mg, 18.5 wt %,) and ethylene glycol dimethad(EGDM, 15 mg 5.1 wt %) are mixed with a solution of PAA-Te **2** (10 mg, 3.4wt %) in DI water (0.2 ml) and deoxygenated by bubbling with nitrogen gas. After adding 2,2'-azobisisobutyronitrile (AIBN, 3 mg, 1.0 wt %) into the mixture (ml), the clear solution is transferred between two glass slides sealed with Teflon. The polymerization is carried out using a UV lamp (320 nm) for 5 hours. In order to prevent possible leaching of PAA-Te **2** from this hydrogel, the resulting hydrogel is further crosslinked by soaking in an excess of 1, 6-diisocyanatohexane overnight at RT. Only a small portion of free amine sites (approx. 10 mol %) of PAA-Te **2** are available to be crosslinked with each other owing to a limited amount of Et3N used during the preparation procedure described above. The resulting interpenetrating network (IPN) hydrogel is thoroughly washed with THF and DI water in order to remove low molecular weight compounds to afford a brown colored hydrogel film 3 (thickness; 0.24mm).

Synthesis of hydrogel **4** (see Scheme 1 (Figure 20B)). Hydrogel **4** is a blank and synthesized by the same methods employed for the preparation of hydrogel **3**. However, PAA hydrochloride is used instead of PAA-Te **2**, and 10 mol % of Et3N equivalent to amine sites in PAA hydrochloride is used during the crosslinking procedure.

### Example 19: Catalytic NO generation by hydrogel

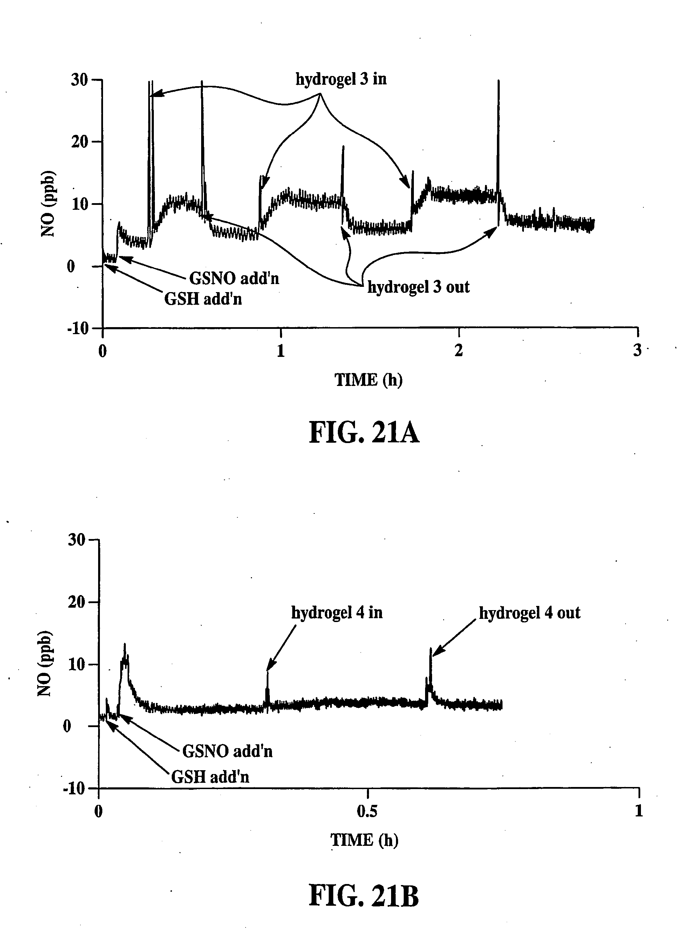

As shown in Figure 21A, hydrogel **3** is capable of catalytically generating NO from GSNO in the presence of GSH in deoxygenated PBS buffer (pH 7.4). A strong copper chelator, EDTA, is added to the test solution in order to capture any small amounts of free copper ion impurities which could cause significant RSNO decomposition. Prior to initiating the experiment, a disk of hydrogel **3** (thickness, 0.24 mm; radius, 0.35 mm) is soaked in PBS buffer (pH 7.4) containing 0.5 mM EDTA. Upon the addition of this hydrogel **3** film into a separate reaction solution containing GSNO and GSH along with EDTA, an increase in NO is detected, eventually reaching steady-state NO flux (see Figure 21A). When hydrogel **3** is removed from reaction cell, the NO flux decreased to nearly the original baseline. Subsequent immersion/removal cycles of the same piece of hydrogel **3** demonstrated almost the same reversible steady-state NO flux. A similar experiment using CySNO and CySH under the same reaction conditions showed that a new piece of the same size hydrogel **3** is also capable of generating NO, suggesting that both small biologically active RSNOs are susceptible to catalytic decomposition by the organotellurium bound polymer.

NO generation of blank hydrogel 4 from GSNO and GSH. To ensure that the NO generation is induced only from organotelluride, not the polymer backbone itself, a blank hydrogel **4** is investigated for catalytic NO generation from GSNO and GSH in deoxygenated PBS buffer (pH 7.4, containing 0.5 mM EDTA) (see Figure 21B). Pre-experiment treatment of hydrogel **4** is the same as the experiments with hydrogel **3**. As shown in Figure 21B, NO generation is not observed upon immersion/removal of similar size hydrogel 4, indicating that the organotelluride is responsible for the NO generation. In general, the compounds of the present disclosure may be prepared by the methods illustrated in the general reaction schemes as, for example, described herein, or by modifications thereof, using readily available starting materials, reagents and conventional synthesis procedures. In these reactions, it is also possible to make use of variants which are in themselves known, but are not mentioned here.

The present disclosure provides among other things, coatings, compositions, devices, and methods. While specific embodiments of the subject disclosure have been discussed, the above specification is illustrative and not restrictive. Many variations of the disclosure will become apparent to those skilled in the art upon review of this specification. The full scope of the disclosure should be determined by reference to the claims.

## Claims

1. A composition for use with a bioimplant, the composition comprising a matrix covalently bound to a chalcogenide moiety; wherein said chalcogenide moiety is selected from an organoselenium moiety and an organotellurium moiety; and wherein the composition decomposes nitrosothiols to generate nitric oxide.

2. The composition of claim 1, wherein said organoselenium moiety is a diselenium moiety.

3. The composition of claim 1, wherein said organotellurium moiety is a ditellurium moiety.

4. The composition of claim 1, wherein said chalcogenide moiety is selected from: an enzyme comprising selenium and an enzyme comprising tellurium.

5. The composition of claim 1, wherein said chalcogenide moiety comprises a moiety selected from structure I or II:
R₁-R₂-R₃-A-R₄ I 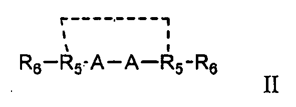
wherein R₁ represents an alkyl, H, aryl, or a bond;
R₂ represents an alkyl, amido, carboxyl, amino, or a bond;
R₃ represents an alkyl or a bond;
A represents independently for each occurrence Se, or Te;
R₄ represents an H, alkyl, or a bond;
the dashed line represents an optional bond included if structure II is cyclic;
R₅ represents independently for each occurrence an alkyl, aryl, amido, carboxyl, amino, or a bond; and
R₆ represents independently for each occurrence an H, carboxyl, amino, aryl, or a bond.

6. The composition of claim 1, wherein the matrix comprises a polymer moiety.

7. The composition of claim 6, wherein said polymer moiety comprises polyurethane, polyester, polyethyleneimine, polymethacrylate, polytetrafluoroethylene, or polydimethylsiloxane.

8. The composition of claim 1, wherein the matrix comprises a fibrous matrix or fumed silica.

9. The composition of claim 1, wherein said matrix further comprises a therapeutic agent.

10. The composition of claim 1, wherein said chalcogenide compound comprises a carboxyl moiety or an amine moiety.

11. The composition of claim 1, wherein said organoselenium moiety is selected from selenocystamine, selenocystine, 3,3'-diselenodipropionic acid, selenocysteine, ebselen, propyl-selenocystine, allyl-selenocystine, methyl-selenocystine, selenomethionine, selenium choline, a diselenium compound, and combinations thereof.

12. The composition of claim 6, wherein said polymer includes one or more of: a carboxyl moiety, an aldehyde moiety, or a halide moiety.

13. The composition of claim 6, wherein said polymer comprises more than about 0.6 mmol/g carboxyl moieties.

14. The composition of claim 6, wherein said polymer is hydrophilic.

15. A biocompatible implantable analyte sensor, comprising:
an electrode surface and disposed thereon an at least partially analyte-permeable composition of any of claims 1-14.

16. The analyte sensor of claim 15, wherein said sensor is subcutaneously implantable.

17. The analyte sensor of claim 15, wherein said sensor is intravascularly implantable.

18. The analyte sensor of claim 15, wherein said sensor detects nitrosothiol.

19. The use of the composition of any one of claims 1 to 14 with a biocompatible implantable sensor of any one of claims 15 to 18, wherein the composition is to be disposed on an electrode surface of the sensor.

## Patentansprüche

1. Zusammensetzung zur Verwendung mit einem Bioimplantat, wobei die Zusammensetzung eine Matrix umfasst, die kovalent an einen Chalkogenid-Teil gebunden ist; wobei der Chalkogenid-Teil aus einem Organoselen-Teil und einem Organotellur-Teil ausgewählt ist und wobei die Zusammensetzung Nitrosothiole zersetzt, um Stickstoffoxid zu erzeugen.

2. Zusammensetzung nach Anspruch 1, wobei es sich bei dem Organoselen-Teil um einen Diselen-Teil handelt.

3. Zusammensetzung nach Anspruch 1, wobei es sich bei dem Organotellur-Teil um einen Ditellur-Teil handelt.

4. Zusammensetzung nach Anspruch 1, wobei der Chalkogenid-Teil aus folgenden ausgewählt ist: einem Enzym, das Selen umfasst, und einem Enzym, das Tellur umfasst.

5. Zusammensetzung nach Anspruch 1, wobei der Chalkogenid-Teil einen Teil umfasst, der aus Struktur I oder II ausgewählt ist:
R₁-R₂-R₃-A-R₄ I 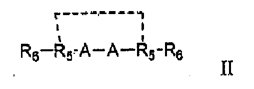
wobei R₁ für ein Alkyl, H, Aryl oder eine Bindung steht;
R₂ für ein Alkyl, Amido, Carboxyl, Amino oder eine Bindung steht;
R₃ für ein Alkyl oder eine Bindung steht;
A unabhängig für jedes Vorkommen für Se oder Te steht;
R₄ für ein H, Alkyl oder eine Bindung steht;
die gestrichelte Linie für eine optionale Bindung steht, die enthalten ist, wenn Struktur II cyclisch ist;
R₅ unabhängig für jedes Vorkommen für ein Alkyl, Aryl, Amido, Carboxyl, Amino oder eine Bindung steht und R₆ unabhängig für jedes Vorkommen für ein H, Carboxyl, Amino, Aryl oder eine Bindung steht.

6. Zusammensetzung nach Anspruch 1, wobei die Matrix einen Polymer-Teil umfasst.

7. Zusammensetzung nach Anspruch 6, wobei der Polymer-Teil Polyurethan, Polyester, Polyethylenimin, Polymethacrylat, Polytetrafluorethylen oder Polydimethylsiloxan umfasst.

8. Zusammensetzung nach Anspruch 1, wobei die Matrix eine Fasermatrix oder Quarzstaub umfasst.

9. Zusammensetzung nach Anspruch 1, wobei die Matrix weiterhin ein Therapeutikum umfasst.

10. Zusammensetzung nach Anspruch 1, wobei die Chalkogenidverbindung einen Carboxyl-Teil oder einen Amin-Teil umfasst.

11. Zusammensetzung nach Anspruch 1, wobei der Organoselen-Teil aus Selenocystamin, Selenocystin, 3,3'-Diselenodipropionsäure, Selenocystein, Ebselen, Propylselenocystin, Allylselenocystin, Methylselenocystin, Selenomethionin, Selencholin, einer Diselenverbindung und Kombinationen davon ausgewählt ist.

12. Zusammensetzung nach Anspruch 6, wobei das Polymer einen oder mehrere der folgenden enthält: einen Carboxyl-Teil, einen Aldehyd-Teil oder einen Halogenid-Teil.

13. Zusammensetzung nach Anspruch 6, wobei das Polymer mehr als etwa 0,6 mmol/g Carboxyl-Teile umfasst.

14. Zusammensetzung nach Anspruch 6, wobei das Polymer hydrophil ist.

15. Biokompatibler implantierbarer Analytsensor, der Folgendes umfasst:
eine Elektrodenoberfläche und darauf angeordnet eine zumindest zum Teil analytendurchlässige Zusammensetzung nach einem der Ansprüche 1 - 14.

16. Analytsensor nach Anspruch 15, wobei der Sensor subkutan implantierbar ist.

17. Analytsensor nach Anspruch 15, wobei der Sensor intravaskulär implantierbar ist.

18. Analytsensor nach Anspruch 15, wobei der Sensor Nitrosothiol erkennt.

19. Verwendung der Zusammensetzung nach einem der Ansprüche 1 bis 14 mit einem biokompatiblen implantierbaren Sensor nach einem der Ansprüche 15 bis 18, wobei die Zusammensetzung auf einer Elektrodenoberfläche des Sensors angeordnet werden soll.

## Revendications

1. Une composition destinée à être utilisée avec une greffe biocompatible, la composition comprend une matrice liée de façon covalente à un groupement chalcogénure ; dans laquelle le groupement chalcogénure mentionné est choisi entre un groupement organosélénium et un groupement organotellure ; et dans laquelle la composition décompose les groupements nitrosothiols pour former de l'oxyde nitrique.

2. La composition de la revendication 1, dans laquelle le groupement organosélénium mentionné est un groupement disélénium.

3. La composition de la revendication 1, dans laquelle le groupement organotellure mentionné est un groupement ditellure.

4. La composition de la revendication 1, dans laquelle le groupement chalcogénure mentionné est choisi entre une enzyme comprenant du sélénium et une enzyme comprenant du tellure.

5. La composition de la revendication 1, dans laquelle le groupement chalcogénure mentionné comprend un groupement choisi de structure 1 ou II :
R₁-R₂-R₃-A-R₄ I 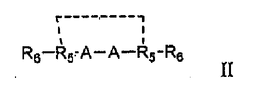
dans laquelle R₁ représente un groupement alkyle, H, aryle, ou une liaison ;
R₂ représente un groupement alkyle, amide, carboxyle, amine, ou une liaison ;
R₃ représente un groupement alkyle ou une liaison ;
A représente de façon indépendante à chaque occurrence du Se ou du Te ;
R₄ représente un groupement H, alkyle, ou une liaison ;
la ligne pointillée représente une liaison optionnelle présente lorsque la structure II est cyclique ;
R₅ représente indépendamment à chaque occurrence un groupement alkyle, aryle, amide, carboxyle, amine, ou une liaison ; et
R₆ représente indépendamment à chaque occurrence un groupement H, carboxyle, amine, aryle, ou une liaison.

6. La composition de la revendication 1, dans laquelle la matrice comprend un groupement polymère.

7. La composition de la revendication 6, dans laquelle le groupement polymère mentionné comprend du polyuréthane, du polyester, de la polyéthylèneimine, du polyméthacrylate, du polytétrafluoroéthylène, ou du polydiméthylsiloxane.

8. La composition de la revendication 1, dans laquelle la matrice comprend une matrice fibreuse ou de la silice pyrogénique.

9. La composition de la revendication 1, dans laquelle la matrice mentionnée comprend aussi un agent thérapeutique.

10. La composition de la revendication 1, dans laquelle le composé chalcogénure mentionné comprend un groupement carboxyle ou un groupement amine.

11. La composition de la revendication 1, dans laquelle le groupement organosélénium mentionné est choisi entre la sélénocystamine, la sélénocystine, l'acide 3,3'-disélénodipropanoïque, la sélénocystéine, l'ebselen, la propyl-sélénocystine, l'allyl-sélénocystine, la méthyl-sélénocystine, la sélénométhionine, le sélénium choline, un composé disélénium, et les combinaisons de ceux-ci.

12. La composition de la revendication 6, dans laquelle le polymère mentionné comprend un ou plusieurs groupements carboxyle, aldéhyde ou halogénure.

13. La composition de la revendication 6, dans laquelle le polymère mentionné comprend plus de 0,6 mmol/g environ de groupements carboxyles.

14. La composition de la revendication 6, dans laquelle le polymère mentionné est hydrophile.

15. Un détecteur d'analytes biocompatible implantable, comprenant :
une surface électrode et une composition de l'une quelconque des revendications 1-14 au moins partiellement perméable aux analytes disposée dessus.

16. Le détecteur d'analytes de la revendication 15, dans lequel le détecteur mentionné est implantable de façon sous-cutanée.

17. Le détecteur d'analytes de la revendication 15, dans lequel le détecteur mentionné est implantable de façon intravasculaire.

18. Le détecteur d'analytes de la revendication 15, dans lequel le détecteur mentionné détecte les groupements nitrosothiols.

19. L'utilisation de la composition de l'une quelconque des revendications 1 à 14 avec un détecteur biocompatible implantable de l'une quelconque des revendications 15 à 18, dans laquelle la composition est destinée à être disposée sur la surface électrode du détecteur.
